(19) Europäisches Patentamt
European Patent Office
Office européen des brevets

(11) Veröffentlichungsnummer: **0 639 575 A1**

(12) # EUROPÄISCHE PATENTANMELDUNG

(21) Anmeldenummer: **94111221.1**

(22) Anmeldetag: **19.07.94**

(51) Int. Cl.[6]: **C07D 471/04**, C07D 498/04, C07D 513/04, A61K 31/55, A61K 31/435, //(C07D513/04, 211:00,277:00),(C07D471/04, 277:00,223:00),(C07D498/04, 263:00,223:00),(C07D471/04, 213:00,235:00),(C07D471/04, 223:00,235:00)

(30) Priorität: **22.07.93 DE 4324580**

(43) Veröffentlichungstag der Anmeldung:
**22.02.95 Patentblatt 95/08**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach (DE)**

(72) Erfinder: **Linz, Günter, Dr.**
**Erlenweg 8**
**D-88441 Mittelbiberach (DE)**
Erfinder: **Himmelsbach, Frank, Dr.**
**Ahornweg 16**
**D-88441 Mittelbiberach (DE)**
Erfinder: **Pieper, Helmut, Dr.**
**Kapellenweg 5**
**D-88400 Biberach (DE)**
Erfinder: **Austel, Volkhard, Prof. Dr.**
**Kapellenweg 7**
**D-88400 Biberach (DE)**
Erfinder: **Müller, Thomas, Dr.**
**Oldenburger Strasse 3**
**D-26188 Edewecht (DE)**
Erfinder: **Weisenberger, Johannes, Dr.**
**Haydnweg 5**
**D-88400 Biberach (DE)**
Erfinder: **Guth, Brian, Dr.**
**Am Schlegelberg 24**
**D-88447 Warthausen (DE)**

(54) **Bicyclische Heterocyclen, diese Verbindungen enthaltende Arzneimittel und Verfahren zu ihrer Herstellung.**

(57) Die Erfindung betrifft bicyclische Heterocyclen der allgemeinen Formel

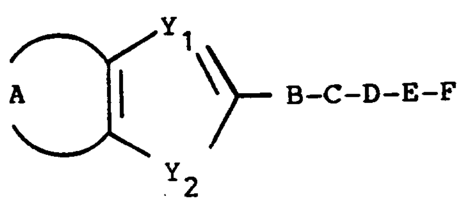

, (I)

in der
A bis F, $Y_1$ und $Y_2$ wie im Anspruch 1 definiert sind, deren Tautomere, deren Stereoisomere einschließlich deren Gemische und deren Salze, insbesondere deren physiologisch verträgliche Salze mit anorganischen oder

EP 0 639 575 A1

organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung sowie neue Zwischenprodukte der allgemeinen Formel Ia.

Die Erfindung betrifft bicyclische Heterocyclen der allgemeinen Formel

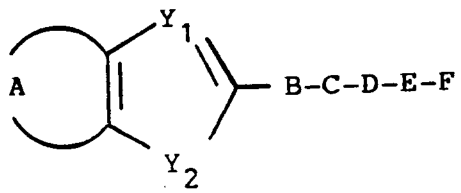

, (I)

deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel und deren Verwendung sowie Verfahren zu ihrer Herstellung.

In der obigen allgemeinen Formel I bedeutet

$Y_1$ ein Stickstoffatom oder ein durch den Rest $R_1$ substituiertes Kohlenstoffatom, wobei

$R_1$ ein Wasserstoffatom oder eine Alkylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine $-N=CH-NR_2-(CH_2)_m-$, $-(CH_2)_m-NR_2-CH=N-$, $-CH=CH-N=CH-$, $-CH=N-CH=CH-$ oder $-(CH_2)_n-NR_2-(CH_2)_p$-Brücke, welche im Kohlenstoffteil jeweils durch eine oder zwei Alkylgruppen substituiert sein kann, wobei

m die Zahl 1 oder 2,

n und p, die gleich oder verschieden sein können, jeweils die Zahl 1, 2 oder 3 und

$R_2$ ein Wasserstoffatom, eine Alkoxycarbonylgruppe mit insgesamt 2 bis 7 Kohlenstoffatomen, eine Alkyl-, Phenylalkyl-, Allyloxycarbonyl-, Phenylalkoxycarbonyl-, Trifluormethylcarbonyl-; $R_1CO$- oder $R_3CO-O-CHR_1-OCO$-Gruppe, wobei $R_1$ wie vorstehend erwähnt definiert ist und

$R_3$ eine Alkylgruppe mit 1 bis 5 Kohlenstoffatomen, eine Cycloalkylgruppe mit 5 bis 7 Kohlenstoffatomen, eine Phenylalkylgruppe oder eine Phenylgruppe bedeutet, darstellen,

B eine gegebenenfalls durch ein Fluor-, Chlor- oder Bromatom, durch eine Cyano-, Trifluormethyl-, Alkyl-, Hydroxy-, Alkoxy-, Alkylsulfenyl-, Alkylsulfinyl- oder Alkylsulfonylgruppe mono- oder disubstituierte Phenylengruppe, wobei die Substituenten gleich oder verschieden sein können und in der zusätzlich 1 oder 2 Methingruppen jeweils durch ein N-Atom ersetzt sein können, oder eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können, oder eine 1,4-Cyclohexylengruppe, in der die Methylengruppe in 2-Stellung oder die Methylengruppen in 2- und 5-Stellung jeweils durch eine $-NR_4$-Gruppe ersetzt sind, wobei in einem so erhaltenen Ring, der ein oder zwei Stickstoffatome oder eine oder zwei $-NR_4$-Gruppen enthält, jeweils eine oder zwei zu einem Stickstoffatom benachbarte Methylengruppen durch eine Carbonylgruppe ersetzt sein können, wobei jedoch jedem Ringstickstoffatom nur eine Carbonylgruppe benachbart sein kann, eine 3,4-Dehydro-1,4-piperidinylen- oder 1,3-Piperidinylengruppe, wobei

$R_4$ ein Wasserstoffatom, eine Alkyl- oder Phenylalkylgruppe darstellt,

C eine $-CH_2-$, $-CH_2CH_2-$, $-CO-$, $-CH_2-SO_2-$ oder $-SO_2-CH_2$-Gruppe,
eine $-CH_2-CO-$ oder $-NR_4-CO$-Gruppe, wobei die Carbonylgruppe jeweils mit dem Rest D verknüpft sowie $R_4$ wie vorstehend erwähnt definiert ist,
eine $-CH_2-NR_4-$, $-NR_4-CH_2-$, $-O-CH_2-$, $-CH_2-O-$, $-S-CH_2-$, $-CH_2-S-$, $-SO-CH_2-$ oder $-CH_2-SO-$, wobei die Sulfinylgruppe, ein Stickstoff-, Sauerstoff- oder Schwefelatom nicht mit einem Stickstoffatom der Reste B oder D direkt oder über ein einzelnes Kohlenstoffatom verbunden sein kann und $R_4$ wie vorstehend erwähnt definiert ist,

D eine 1,4-Cyclohexylen- oder 1,4-Cyclohex-3-enylengruppe, in denen eine oder beide >CH-Einheiten, die mit den Resten C, E oder F verknüpft sind, durch ein Stickstoffatom ersetzt sein können und die >CH-Einheit, die mit dem Rest E oder F verknüpft ist, auch durch eine >C=CH-Gruppe ersetzt sein kann, oder eine $-NR_5$-X-Gruppe, in der

X eine geradkettige oder verzweigte Alkylengruppe mit 1 bis 5 Kohlenstoffatomen oder eine 1,4-Cyclohexylengruppe und

$R_5$ ein Wasserstoffatom, eine Phenylalkylgruppe mit 1 bis 4 Kohlenstoffatomen im Alkylteil oder eine Alkylgruppe mit 1 bis 6 Kohlenstoffatomen, welche in 1-, 2- oder 3-Stellung durch eine Morpholinocarbonyl-,

Pyrrolidinocarbonyl-, Piperidinocarbonyl- oder Hexamethyleniminocarbonylgruppe oder durch eine ($R_6NR_7$)-CO-Gruppe substituiert sein kann, darstellen, wobei

$R_6$ ein Wasserstoffatom, eine Alkylgruppe mit 1 bis 5 Kohlenstoffatomen, eine Cycloalkylgruppe mit 5 bis 7 Kohlenstoffatomen, eine Phenyl- oder Phenylalkylgruppe und

$R_7$ ein Wasserstoffatom, eine Alkylgruppe mit 1 bis 4 Kohlenstoffatomen, eine Cyloalkylgruppe mit 5 bis 7 Kohlenstoffatomen oder eine Phenylgruppe darstellen,

E eine Alkylengruppe oder auch, falls D nicht über ein N-Atom mit dem Rest F direkt verbunden ist, eine Bindung und

F eine durch eine $R_8O$-Gruppe substituierte Carbonylgruppe, in der

$R_8$ ein Wasserstoffatom, eine Alkylgruppe mit 1 bis 6 Kohlenstoffatomen, die in 1-, 2-, 3-Stellung durch eine Morpholinocarbonyl-, Pyrrolidinocarbonyl-, Piperidinocarbonyl-, Hexamethyleniminocarbonyl- oder ($R_6NR_7$)-CO-Gruppe, wobei $R_6$ und $R_7$ wie vorstehend erwähnt definiert sind, oder in 2- oder 3-Stellung durch eine Morpholino- oder Pyrrolidinon-1-yl-Gruppe substituiert sein kann, eine Phenylalkyl- oder Pyridylalkylgruppe, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe mit 1 bis 4 Kohlenstoffatomen oder durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, eine Indanyl- oder Cinnamylgruppe darstellt,

eine Phosphono-, O-Alkylphosphono- oder $R_9CO\text{-}O\text{-}CHR_6\text{-}O\text{-}CO$-Gruppe darstellen, wobei

$R_6$ wie vorstehend erwähnt definiert ist und

$R_9$ eine Alkylgruppe mit 1 bis 5 Kohlenstoffatomen, eine Cycloalkyl- oder Cycloalkoxygruppe mit jeweils 5 bis 7 Kohlenstoffatomen im Cycloalkylteil, eine Alkoxygruppe mit 1 bis 4 Kohlenstoffatomen, eine Phenyl-, Phenylalkyl- oder Phenylalkoxygruppe darstellt,

wobei, soweit nichts anderes erwähnt wurde,

die vorstehend erwähnten Alkyl-, Alkylen- oder Alkoxyteile jeweils 1 bis 3 Kohlenstoffatome enthalten können.

Ein weiterer Gegenstand der vorliegenden Erfindung sind die neuen Zwischenprodukte der allgemeinen Formel

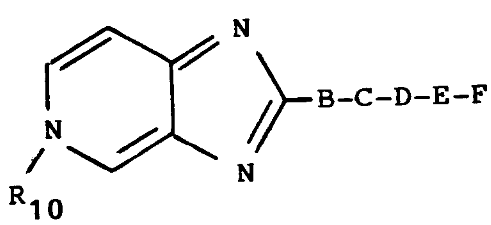

, (Ia)

in der

B bis F wie vorstehend erwähnt definiert sind und

$R_{10}$ eine Alkyl- oder Phenylalkylgruppe mit jeweils 1 bis 3 Kohlenstoffatomen im Alkylteil bedeutet, und deren Salze.

Bevorzugte Verbindungen der obigen allgemeinen Formel I sind diejenigen, in denen

$Y_1$ ein Stickstoffatom oder ein durch den Rest $R_1$ substituiertes Kohlenstoffatom, wobei

$R_1$ ein Wasserstoffatom oder eine Alkylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine -CH = CH-N = CH-, -CH = N-CH = CH- oder $-(CH_2)_n-NR_2-(CH_2)_p$-Brücke, in der

n und p, die gleich oder verschieden sein können, jeweils die Zahl 1, 2 oder 3 und

$R_2$ ein Wasserstoffatom, eine Alkoxycarbonylgruppe mit insgesamt 2 bis 6 Kohlenstoffatomen, eine Alkyl-, Allyloxycarbonyl- oder Trifluormethylcarbonylgruppe bedeutet, darstellen,

B eine gegebenenfalls durch ein Fluor-, Chlor- oder Bromatom, durch eine Alkyl-, Trifluormethyl- oder Alkoxygruppe mono- oder disubstituierte Phenylengruppe, wobei die Substituenten gleich oder verschieden sein können und in der zusätzlich 1 oder 2 Methingruppen jeweils durch ein N-Atom ersetzt sein können, oder

eine 1,4-Cyclohexylengruppe, in der eine >CH-Einheit in 1- oder 4-Stellung durch ein Stickstoffatom oder

die >CH-Einheiten in 1- und 4-Stellung jeweils durch ein Stickstoffatom ersetzt sein können, eine 3,4-Dehydro-1,4-piperidinylen- oder 1,3-Piperidinylengruppe,

C eine $-CH_2-$, $-CH_2CH_2-$, -CO-, $-CH_2-SO_2-$ oder $-SO_2-CH_2$-Gruppe, eine $-CH_2$-CO- oder $-NR_4$-CO-Gruppe, wobei die Carbonylgruppe jeweils mit dem Rest D verknüpft und

$R_4$ ein Wasserstoffatom oder eine Alkylgruppe darstellt, eine $-CH_2-NR_4-$, $-NR_4-CH_2-$, $-O-CH_2-$, $-CH_2-O-$, $-S-CH_2-$, $-CH_2-S-$, $-SO-CH_2-$ oder $-CH_2-SO-$, wobei die Sulfinylgruppe, ein Stickstoff-, Sauerstoff- oder Schwefelatom nicht mit einem Stickstoffatom der Reste B oder D direkt oder über ein Kohlenstoffatom verbunden sein kann und $R_4$ wie vorstehend erwähnt definiert ist,

D eine 1,4-Cyclohexylen- oder 1,4-Cyclohex-3-enylengruppe, in denen die >CH-Einheit in 1-Stellung, welche mit dem Rest C verknüpft ist, oder die >CH-Einheit in 4-Stellung, welche mit dem Rest E oder F verknüpft ist, oder auch beide >CH-Einheiten durch ein Stickstoffatom ersetzt sein können, wobei die >CH-Einheit in 4-Stellung auch durch eine >C=CH-Gruppe ersetzt sein kann, oder

eine $-NR_5$-X-Gruppe, in der

X eine geradkettige oder verzweigte Alkylengruppe mit 1 bis 5 Kohlenstoffatomen oder eine 1,4-Cyclohexylengruppe und

$R_5$ ein Wasserstoffatom, eine Alkyl- oder Phenylalkylgruppe darstellen,

E eine Alkylengruppe oder auch, falls D nicht über ein N-Atom mit dem Rest F direkt verknüpft ist, eine Bindung und

F eine durch eine $R_8O$-Gruppe substituierte Carbonylgruppe, in der

$R_8$ ein Wasserstoffatom, eine Alkylgruppe mit 1 bis 6 Kohlenstoffatomen, die in 1-, 2-, 3-Stellung durch eine Phenyl- oder Pyridylgruppe oder in 2- oder 3-Stellung durch eine Morpholinogruppe substituiert sein kann, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils der Cycloalkylteil durch eine Alkylgruppe mit 1 bis 4 Kohlenstoffatomen oder durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, eine Indanyl- oder Cinnamylgruppe darstellt,

oder eine $R_9CO-O-CHR_6-O-CO$-Gruppe darstellt, in der

$R_6$ ein Wasserstoffatom, eine Alkylgruppe, eine Phenylgruppe oder Cycloalkylgruppe mit 5 bis 7 Kohlenstoffatomen und

$R_9$ eine Alkylgruppe mit 1 bis 5 Kohlenstoffatomen, eine Alkoxygruppe mit 1 bis 4 Kohlenstoffatomen, eine Phenyl- oder Cycloalkoxygruppe mit 5 bis 7 Kohlenstoffatomen darstellen,

deren Tautomere, deren Stereoisomere einschließlich deren Gemische und deren Salze, und

die neuen Zwischenprodukte der allgemeinen Formel (Ia), in der

B bis F wie vorstehend erwähnt definiert sind und

$R_{10}$ eine Alkylgruppe darstellt,

wobei, soweit nichts anderes erwähnt wurde,

die vorstehend erwähnten Alkyl-, Alkylen- oder Alkoxyteile jeweils 1 bis 3 Kohlenstoffatome enthalten können.

Besonders bevorzugte Verbindungen der obigen allgemeinen Formel I sind diejenigen, in denen

$Y_1$ ein Stickstoffatom oder ein durch den Rest $R_1$ substituiertes Kohlenstoffatom, wobei

$R_1$ ein Wasserstoffatom, eine Methyl- oder Ethylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine -CH=CH-N=CH-, -CH=N-CH=CH-, $-(CH_2)_2-HR_2-CH_2-$ oder $-(CH_2)_2-NR_2-(CH_2)_2$-Brücke, in der

$R_2$ ein Wasserstoffatom, eine Methyl-, Ethyl-, Allyloxycarbonyl- oder Trifluormethylcarbonylgruppe oder eine Alkoxycarbonylgruppe mit insgesamt 2 bis 5 Kohlenstoffatomen darstellt,

B eine gegebenenfalls durch ein Fluor-, Chlor- oder Bromatom, durch eine Methyl- oder Ethylgruppe substituierte Phenylengruppe, in der zusätzlich eine Methingruppe durch ein N-Atom ersetzt sein kann, eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können, oder eine 1,3-Piperidinylengruppe,

C eine $-CH_2-$, $-CH_2CH_2-$ oder -CO-Gruppe,

eine $-CH_2$-CO- oder $-NR_4$-CO-Gruppe, wobei die Carbonylgruppe jeweils mit dem Rest D verknüpft und

$R_4$ ein Wasserstoffatom oder eine Alkylgruppe mit 1 oder 2 Kohlenstoffatomen darstellt,

eine $-CH_2-NR_4-$, $-NR_4-CH_2-$, $-O-CH_2-$ oder $-CH_2-O$-Gruppe, wobei das Stickstoff- oder Sauerstoffatom nicht mit einem Stickstoffatom der Reste B oder D direkt oder über ein einzelnes Kohlenstoffatom verbunden sein kann und $R_4$ wie vorstehend erwähnt definiert ist,

D eine 1,4-Cyclohexylengruppe, in der die >CH-Einheit in 1-Stellung, welche mit dem Rest C verknüpft ist, durch ein Stickstoffatom ersetzt sein kann, oder
eine $-NR_5$-1,4-cyclohexylen-Gruppe, in der
$R_5$ ein Wasserstoffatom, eine Methyl-, Ethyl- oder Benzylgruppe darstellt,
E eine Alkylengruppe mit 1 bis 3 Kohlenstoffatomen oder eine Bindung und
F eine durch eine $R_8O$-Gruppe substituierte Carbonylgruppe oder eine $R_9CO-O-CHR_6-O-CO$-Gruppe bedeuten, in denen
$R_6$ ein Wasserstoffatom oder eine Methylgruppe,
$R_8$ ein Wasserstoffatom oder eine Alkylgruppe mit 1 bis 5 Kohlenstoffatomen und
$R_9$ eine Alkylgruppe mit 1 bis 5 Kohlenstoffatomen, eine Methoxy- oder Ethoxygruppe darstellen, und
die neuen Zwischenprodukte der allgemeinen Formel (Ia), in der
B bis F wie vorstehend erwähnt definiert sind und
$R_{10}$ eine Methylgruppe darstellt,
insbesondere diejenigen Verbindungen der allgemeinen Formel I, in denen
$Y_1$ ein Stickstoffatom,
$Y_2$ eine -NH-Gruppe oder ein Schwefelatom,
A eine $-CH=CH-N=CH-$, $-(CH_2)_2-NR_2-CH_2-$ oder $-(CH_2)_2-NR_2-(CH_2)_2$-Brücke, in der
$R_2$ ein Wasserstoffatom, eine Methyl-, Ethyl- oder Trifluormethylcarbonylgruppe oder eine Alkoxycarbonylgruppe mit insgesamt 2 bis 5 Kohlenstoffatomen darstellt,
B eine gegebenenfalls durch eine Methylgruppe substituierte Phenylengruppe oder
eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können,
C eine -CO-Gruppe,
D eine 1,4-Cyclohexylengruppe, in der die >CH-Einheit in 1-Stellung, welche mit dem Rest C verknüpft ist, durch ein Stickstoffatom ersetzt sein kann, oder
eine $-NR_5$-1,4-cyclohexylen-Gruppe, in der
$R_5$ ein Wasserstoffatom oder eine Methylgruppe darstellt,
E eine Methylengruppe oder eine Bindung und
F eine durch eine $R_8O$-Gruppe substituierte Carbonylgruppe, in der
$R_8$ ein Wasserstoffatom oder eine Alkylgruppe mit 1 bis 5 Kohlenstoffatomen darstellt,
bedeuten,
deren Tautomere, deren Stereoisomere einschließlich deren Gemische und deren Salze.

Als besonders bevorzugte Verbindungen seien folgende erwähnt:

(a) 2-[4-[(trans-4-Carboxy-cyclohexyl)-aminocarbonyl]-phenyl]-4,5,6,7-tetrahydro-thiazolo[5,4-c]pyridin,
(b) 2-[4-[(trans-4-Methoxycarbonyl-cyclohexyl)-aminocarbonyl]-phenyl]-4,5,6,7-tetrahydro-thiazolo[5,4-c]-pyridin,
(c) 2-[4-[(trans-4-Carboxy-cyclohexyl)-aminocarbonyl]-phenyl]-4,5,7,8-tetrahydro-6H-thiazolo[4,5-d]azepin,
(d) 2-[4-[(trans-4-Methoxycarbonyl-cyclohexyl)-aminocarbonyl]-phenyl]-4,5,7,8-tetrahydro-6H-thiazolo[4,5-d]azepin,
(e) 2-[1-[(trans-4-Carboxy-cyclohexyl)-aminocarbonyl]-piperid-4-yl]-4,5,6,7-tetrahydro-thiazolo[5,4-c]-pyridin,
(f) 2-[1-[(trans-4-Carboxy-cyclohexyl)-aminocarbonyl]-piperid-4-yl]-4,5,7,8-tetrahydro-6H-thiazolo[4,5-d]-azepin,
(g) 2-[1-[(trans-4-Methoxycarbonyl-cyclohexyl)-aminocarbonyl]-piperid-4-yl]-4,5,7,8-tetrahydro-6H-thiazolo[4,5-d]azepin,
(h) 2-[1-[(trans-4-Isobutyloxycarbonyl-cyclohexyl)-aminocarbonyl]-piperid-4-yl]-4,5,7,8-tetrahydro-6H-thiazolo[4,5-d]azepin,
(i) 2-[4-[(trans-4-Carboxy-cyclohexyl)-aminocarbonyl]-2-methyl-phenyl]-4,5,6,7-tetrahydro-thiazolo[5,4-c]-pyridin,
(j) 2-[4-[(trans-4-Methoxycarbonyl-cyclohexyl)-aminocarbonyl]-2-methyl-phenyl]-4,5,6,7-tetrahydro-thiazolo[5,4-c]pyridin,
(k) 2-[trans-4-[(trans-4-Carboxy-cyclohexyl)-aminocarbonyl]-cyclohexyl]-4,5,7,8-tetrahydro-6H-thiazolo[4,5-d]azepin,
(1) 2-[1-[(trans-4-Ethoxycarbonyl-cyclohexyl)-aminocarbonyl]-piperid-4-yl]-4,5,7,8-tetrahydro-6H-thiazolo-[4,5-d]azepin,
(m) 2-[1-[(trans-4-Isopropoxycarbonyl-cyclohexyl)-aminocarbonyl]-piperid-4-yl]-4,5,7,8-tetrahydro-6H-thiazolo[4,5-d]azepin und
deren Salze.

Die neuen Verbindungen lassen sich beispielsweise nach folgenden Verfahren herstellen:

a) Zur Herstellung von Verbindungen der allgemeinen Formel I, in der $R_2$ ein Wasserstoffatom oder F eine Carboxygruppe oder $R_2$ ein Wasserstoffatom und F eine Carboxygruppe darstellen:

Umwandlung einer Verbindung der allgemeinen Formel

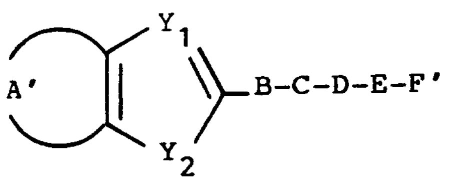

, (II)

in der

B bis E, $Y_1$ und $Y_2$ wie eingangs definiert sind,

A' die für A eingangs erwähnten Bedeutungen besitzt, wobei jedoch zusätzlich $R_2$ einen mittels Hydrolyse, Behandeln mit einer Säure oder Base, Thermolyse oder Hydrogenolyse abspaltbaren Rest darstellt, und F' eine Carboxygruppe oder eine mittels Hydrolyse, Behandeln mit einer Säure, Thermolyse oder Hydrogenolyse in eine Carboxygruppe überführbare Gruppe bedeutet, wobei jedoch mindestens einer der Reste A' oder F' eine mittels Hydrolyse, Behandeln mit Säure, Thermolyse oder Hydrogenolyse abspaltbaren Rest enthalten muß, in eine Verbindung der allgemeinen Formel I, in der $R_2$ ein Wasserstoffatom oder F eine Carboxylgruppe oder $R_2$ ein Wasserstoffatom und F eine Carboxygruppe darstellen.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe und Ester mit Aralkanolen, z.B. der Benzylester, mittels Hydrogenolyse in eine Carboxylgruppe sowie Iminogruppen, die durch einen Schutzrest wie durch die Formyl-, Acetyl-, Trifluoracetyl-, Allyloxycarbonyl-, Ethoxycarbonyl-, tert.Butoxycarbonyl- oder Benzyloxycarbonylgruppe substituiert sind, mittels Hydrolyse, Iminogruppen, die durch einen Schutzrest wie durch die tert.-Butoxycarbonyl-, Benzyloxycarbonyl-, Benzyl-, Methoxybenzyl- oder 2,4-Dimethoxybenzylgruppe substituiert sind, mittels Hydrogenolyse und Iminogruppen, die durch einen Schutzrest wie die Allyloxycarbonylgruppe substituiert sind, in Gegenwart eines Katalysators wie Tetrakis-(triphenylphosphin)-palladium(O) in eine freie Iminogruppe übergeführt werden.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemische oder in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Enthält A' oder bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Enthält A' oder bedeutet F' in einer Verbindung der Formel II beispielsweise die Benzyloxycarbonylgruppe, so kann die Benzylgruppe auch hydrogenolytisch in Gegenwart eines Hydrierungskatalysators

wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden.

Enthält A' eine Allyloxycarbonylgruppe in einer Verbindung der allgemeinen Formel II, so erfolgt die Abspaltung der Allyloxycarbonylgruppe durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100 ° C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo-[2.2.2]octan bei Temperaturen zwischen 20 und 70 ° C.

b) Zur Herstellung von Verbindungen der allgemeinen Formel I, in der C eine -CO- oder $-CH_2CO-$Gruppe darstellt:

Umsetzung einer Verbindung der allgemeinen Formel

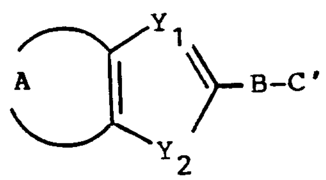

, (III)

in der

A, B, $Y_1$ und $Y_2$ wie eingangs definiert sind und

C' eine Carboxy- oder Carboxymethylgruppe darstellt, mit einem Amin der allgemeinen Formel

H-D-E-F ,(IV)

in der

D bis F wie eingangs definiert sind.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, Dimethylaminopyridin oder 1-Hydroxybenzotriazol, N,N'-Carbonyldiimidazol- oder N,N'-Thionyldiimidazol oder Triphenylphosphin/Tetrachlorkohlenstoff, zweckmäßigerweise bei Temperaturen zwischen 0 und 150 ° C, vorzugsweise bei Temperaturen zwischen 0 und 50 ° C, durchgeführt.

c) Zur Herstellung von Verbindungen der allgemeinen Formel I, in der C eine -CO- oder $-NR_4CO-$Gruppe darstellt:

Umsetzung einer Verbindung der allgemeinen Formel

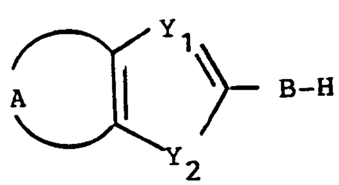

, (V)

in der

A, B, $Y_1$ und $Y_2$ wie eingangs definiert sind, mit einer Verbindung der allgemeinen Formel

$Z_1$-C''-D-E-F ,(VI)

in der

D bis F wie eingangs definiert sind,
C" eine Carbonylgruppe und
$Z_1$ eine Austrittsgruppe wie ein Halogenatom, z. B. ein Chlor- oder Bromatom, oder eine gegebenenfalls substituierte Phenoxygruppe, z. B. eine p-Nitro-phenoxygruppe, darstellen. Die Umsetzung wird zweckmäßigerweise in einem Lösungsmittel oder Lösungsmittelgemisch wie Wasser, Tetrahydrofuran, Tetrahydrofuran/Wasser, Dioxan, Dioxan/Wasser, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 150 °C, vorzugsweise jedoch bei Temperaturen zwischen -10 und 120 °C, durchgeführt.

d) Zur Herstellung von Verbindungen der allgemeinen Formel I, in der F eine durch eine $R_8$'O-Gruppe substituierte Carbonylgruppe darstellt, wobei $R_8$' mit Ausnahme des Wasserstoffatoms die für $R_8$ eingangs erwähnten Bedeutungen besitzt:
Umsetzung einer Verbindung der allgemeinen Formel

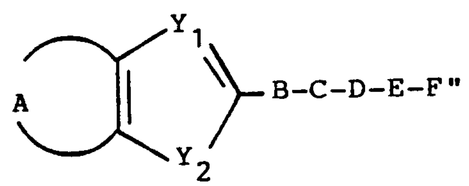

, (VII)

in der
A bis E, $Y_1$ und $Y_2$ wie eingangs definiert sind und
F" eine Carboxy- oder Alkoxycarbonylgruppe darstellt, mit einem Alkohol der allgemeinen Formel

HO - $R_8$' ,(VIII)

in der
$R_8$' mit Ausnahme des Wasserstoffatoms die für $R_8$ eingangs erwähnten Bedeutungen besitzt.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, Dimethylaminopyridin oder 1-Hydroxybenzotriazol, N,N'-Carbonyldiimidazol- oder N,N'-Thionyldiimidazol oder Triphenylphosphin/Tetrachlorkohlenstoff, zweckmäßigerweise bei Temperaturen zwischen 0 und 150 °C, vorzugsweise bei Temperaturen zwischen 0 und 50 °C, durchgeführt.

Die Umsetzung einer entsprechenden Alkoxyverbindung der allgemeinen Formel VII mit einem Alkohol der allgemeinen Formel X wird vorzugsweise in dem betreffenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 100 °C, vorzugsweise bei Temperaturen zwischen 20 und 80 °C, durchgeführt.

e) Zur Herstellung von Verbindungen der allgemeinen Formel I, in der F eine durch eine $R_8$'O-Gruppe substituierte Carbonylgruppe oder eine $R_9$CO-O-CH$R_6$-O-CO-Gruppe darstellt, wobei $R_6$ und $R_9$ wie eingangs erwähnt definiert sind und $R_8$' mit Ausnahme des Wasserstoffatoms die für $R_8$ eingangs erwähnten Bedeutungen besitzt:
Umsetzung einer Verbindung der allgemeinen Formel

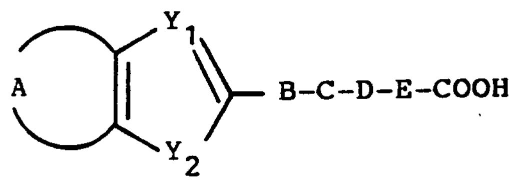

, (IX)

in der
A bis E, $Y_1$ und $Y_2$ wie eingangs definiert sind, mit einer Verbindung der allgemeinen Formel

$Z_2$ - $R_{11}$ ,(x)

in der
$R_{11}$ mit Ausnahme des Wasserstoffatoms die für $R_8$ eingangs erwähnten Bedeutungen besitzt oder eine $R_9CO$-O-$CHR_6$-Gruppe darstellt, wobei $R_6$ und $R_9$ wie eingangs erwähnt definiert sind und
$Z_2$ eine Austrittsgruppe wie ein Halogenatom, z. B. ein Chlor- oder Bromatom, darstellt.

Die Umsetzung wird zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100 °C, vorzugsweise jedoch bei Temperaturen zwischen -10 und 80 °C, durchgeführt.

f) Zur Herstellung von Verbindungen der allgemeinen Formel I, in der $R_2$ eine Alkoxycarbonylgruppe mit insgesamt 2 bis 5 Kohlenstoffatomen, eine Alkyl-, Phenylalkyl-, Allyloxycarbonyl-, Phenylalkoxycarbonyl-, Trifluormethylcarbonyl-, $R_1CO$- oder $R_3CO$-O-$CHR_1$-OCO-Gruppe darstellt:
Umsetzung einer Verbindung der allgemeinen Formel

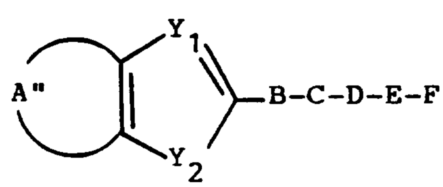

, (XI)

in der
B bis F, $Y_1$ und $Y_2$ wie eingangs definiert sind und
A" eine -N=CH-NH-$(CH_2)_m$-, -$CH_2$-NH-CH=N- oder $(CH_2)_n$-NH-$(CH_2)_p$-Brücke, welche im Kohlenstoffteil jeweils durch eine oder zwei Alkylgruppen substituiert sein kann, wobei p, m und n wie eingangs definiert sind, mit einer Verbindung der allgemeinen Formel

$Z_3$-$R_2$' ,(XII)

in der
$R_2$' mit Ausnahme des Wasserstoffatoms die für $R_2$ eingangs erwähnten Bedeutungen besitzt und
$Z_3$ eine Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe, oder $Z_3$ zusammen mit einem benachbarten Wasserstoffatom des Restes $R_2$' ein Sauerstoffatom bedeuten.

Die Umsetzung wird vorzugsweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumkarbonat, Kaliumkarbonat oder Natronlauge oder in Gegenwart einer tertiären organischen Base wie N-Ethyl-diisopropylamin oder N-Methylmorpholin, welche gleichzeitig auch als Lösungsmittel dienen können, oder gegebenenfalls in Gegenwart von Silberkarbonat oder Silberoxid bei Temperaturen zwischen

-30 und 100 °C, vorzugsweise jedoch bei Temperaturen zwischen -10 und 80 °C, durchgeführt.

Die Alkylierung mit einer Verbindung der Formel XII, in der $Z_3$ eine Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

Die reduktive Alkylierung mit einer Carbonylverbindung der allgemeinen Formel XII wird in Gegenwart eines komplexen Metallhydrids wie Natriumborhydrid, Lithiumborhydrid oder Natriumcyanborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck 1 bis 5 bar durchgeführt. Die Methylierung wird jedoch vorzugsweise in Gegenwart von Ameisensäure als Reduktionsmittel bei erhöhten Temperaturen, z. B. bei Temperaturen zwischen 60 und 120 °C, durchgeführt.

Die Acylierung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 60 °C, durchgeführt.

g) Zur Herstellung von Verbindungen der allgemeinen Formel I, in der A eine $-CH_2-NR_2-(CH_2)_2-$Brücke, in der $R_2$ ein Wasserstoffatom darstellt oder die für $R_{10}$ eingangs erwähnten Bedeutungen besitzt und die im Kohlenstoffteil durch eine oder zwei Alkylgruppen substituiert sein kann, einer der Reste $Y_1$ oder $Y_2$ ein Stickstoffatom und der andere der Reste $Y_1$ oder $Y_2$ eine Iminogruppe darstellen:

Hydrierung einer Verbindung der allgemeinen Formel

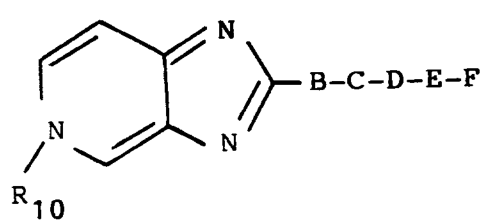

, (Ia)

in der

B bis F und $R_{10}$ wie eingangs definiert sind.

Die katalytische Hydrierung wird vorzugsweise in einem geeigneten Lösungsmittel wie Methanol, Methanol/Wasser, Essigsäure, Essigester, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin, Rhodium oder Palladium/Kohle, bei Temperaturen zwischen 0 und 100 °C, vorzugsweise bei Temperaturen zwischen 20 und 80 °C, durchgeführt.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino-, Imino- oder Amidinogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe,

als Schutzreste für eine Phosphonogruppe eine Alkylgruppe wie die Methyl-, Ethyl-, Isopropyl- oder n-Butylgruppe, die Phenyl- oder Benzylgruppe,

als Schutzrest für eine gegebenenfalls durch eine Alkylgruppe substituierte Amidinogruppe die Benzyloxycarbonylgruppe und

als Schutzrest für eine Amino-, Alkylamino- oder Iminogruppe die Formyl-, Acetyl-, Trifluoracetyl-, Allyloxycarbonyl-, Ethoxycarbonyl-, tert.Butoxycarbonyl-, Benzyloxycarbonyl-, Benzyl-, Methoxy- benzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 120 °C, vorzugsweise bei Temperaturen zwischen 10 und 100 °C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 100 °C, vorzugsweise jedoch bei Temperaturen zwischen 20 und 60 °C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Ether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120 °C oder durch Behandlung mit Natronlauge oder wäßriger Lithiumhydroxid-Lösung gegebenenfalls in Gegenwart eines Lösungsmittels wie Tetrahydrofuran oder Methanol bei Temperaturen zwischen 0 und 50 °C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Allylgruppenakzeptors wie Morpholin oder 1,3-Dimedon bei Temperaturen zwischen 0 und 100 °C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wäßrigem Ethanol und gegebenenfalls in Gegenwart einer Base wie 1,4-Diazabicyclo[2.2.2]octan bei Temperaturen zwischen 20 und 70 °C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 °C.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei Temperaturen zwischen 40 und 150 °C, vorzugsweise jedoch bei Temperaturen zwischen 60 und 100 °C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen O °C und der Siedetemperatur des Reaktionsgemisches, vorzugsweise jedoch bei Temperaturen zwischen 20 und 60 °C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalisch chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren

sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren (siehe Beispiele), beispielsweise durch Cyclisierung eines entsprechenden o-Pyridyldiamins mit einem entsprechenden Carbonsäurederivat oder durch Umsetzung eines entsprechenden Carbonsäurederivates mit einem entsprechenden 3-Halogen-piperid-4-on oder 5-Halogen-azepin-4-on. Ein so erhaltenes bicyclisches Derivat kann anschließend mittels Hydrolyse, Alkylierung und/oder Acylierung in eine gewünschte Ausgangsverbindung übergeführt werden.

Die Herstellung einer Verbindung der allgemeinen Formel Ia erfolgt durch Alkylierung eines entsprechenden Benzimidazols der allgemeinen Formel I mit einem entsprechenden Alkyl- oder Phenylalkylhalogenid wie Methyljodid oder Benzylbromid in einem Lösungsmittel wie Dimethylsulfoxid und in Gegenwart einer Base wie Kalium-tert.butylat bei Temperaturen zwischen 0 und 50°C, vorzugsweise bei Raumtemperatur.

Wie bereits eingangs erwähnt, weisen die neuen bicyclischen Heterocyclen der allgemeinen Formel I und deren Salze, insbesondere deren physiologisch verträgliche Salze mit anorganischen oder organischen Säuren oder Basen, wertvolle Eigenschaften auf. So weisen die neuen Verbindungen der allgemeinen Formel I, in denen A eine basische Gruppe oder eine gegebenenfalls in vivo in eine basische Gruppe überführbare Gruppe enthält und F eine Carboxyl-, Phosphono oder O-Alkylphosphonogruppe oder eine gegebenenfalls in vivo in eine Carboxyl-, Phosphono- oder O-Alkyl-phosphonogruppe überführbare Gruppe, z.B. eine durch eine Alkoxy- oder Cycloalkoxygruppe substituierte Carbonylgruppe, darstellt, wertvolle pharmakologische Eigenschaften auf, neben einer entzündungshemmenden und den Knochenabbau hemmenden Wirkung insbesondere antithrombotische, antiaggregatorische und tumor bzw. metastasenhemmende Wirkungen.

Beispielsweise wurden die Verbindungen der allgemeinen Formel I auf ihre biologischen Wirkungen wie folgt untersucht:

1. Hemmung der Bindung von $^{3}$H-BIBU 52 an Humanthrombozyten:

Eine Suspension von Humanthrombozyten in Plasma wird mit $^{3}$H-BIBU 52 [= (3S,5S)-5-[(4'-Amidino-4-biphenylyl)oxymethyl]-3-[(carboxyl)methyl]-2-pyrrolidinon[3-$^{3}$H-4-biphenylyl]], das den literaturbekannten Liganden $^{125}$J-Fibrinogen ersetzt, (siehe deutsche Patentanmeldung P 42 14 245.8 der gleichen Anmelderin vom 30. 04. 1992, internes Zeichen: Case 5/1093-FL) und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^{3}$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^{3}$H-BIBU 52 (Endkonzentration: 5 nM) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene

Restplasma wird aus dem $^{14}C$-Gehalt bestimmt, der gebundene Ligand aus der $^{3}H$-Messung. Nach Abzug der unspezifischen Bindung wird die Pelletaktivität gegen die Konzentration der Testsubstanz aufgetragen und die Konzentration für eine 50°ige Hemmung der Bindung ermittelt.

2. Antithrombotische Wirkung:

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 2(1) | 210 | 2600 |
| 2(4) | 450 | 1440 |
| 3(5) | 31 | 100 |
| 3(6) | 42 | 100 |
| 3(8) | 2100 | 4200 |
| 3(12) | 2000 | 2900 |
| 4 | 96 | 410 |
| 4(1) | 75 | 680 |
| 4(3) | 24 | 220 |
| 4(4) | 120 | 110 |
| 7 | 1600 | 660 |
| 7(1) | 1400 | 1400 |
| 7(2) | 1300 | 2600 |
| 7(3) | 1500 | 1300 |

Die erfindungsgemäßen Verbindungen sind gut verträglich, da beispielsweise nach intravenöser Gabe von 30 mg/kg der Verbindungen der Beispiele 4 und 4(1) an der Maus keines der 3 getesteten Tiere verstarb.

Auf Grund ihrer Hemmwirkung auf Zell-Zell- bzw. Zell-Matrix-Wechselwirkungen eignen sich die neuen cyclischen Harnstoffderivate der allgemeinen Formel I und ihre physiologisch verträglichen Salze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie ThromboxanRezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

Herstellung der Ausgangsverbindungen:

Beispiel I

4-(Aminothiocarbonyl)-benzoesäure-ethylester

Durch eine Lösung von 15,0 g 4-Cyan-benzoesäure-ethylester in 100 ml Pyridin und 12 ml Triethylamin leitet man 2 Stunden Schwefelwasserstoff. Nach zweistündigem Rühren bei Raumtemperatur wird nochmals eine Stunde Schwefelwasserstoff eingeleitet. Man rührt 2 Tage bei Raumtemperatur. Die Lösung wird mit einem Liter Wasser verdünnt und der Niederschlag abgesaugt und getrocknet.

Ausbeute: 16,3 g (91 % der Theorie),
$R_f$-Wert: 0,36 (Kieselgel; Methylenchlorid/Methanol = 19:1)

Analog Beispiel I wird folgende Verbindung erhalten:

(1) 3-(Aminothiocarbonyl)-benzoesäure-methylester $R_f$-Wert: 0,65 (Kieselgel; Essigester/Cyclohexan = 1:1)
(2) 4-(Aminothiocarbonyl)-3-methyl-benzoesäure-methylester $R_f$-Wert: 0,56 (Kieselgel; Essigester/Cyclohexan = 1:1)
(3) trans-4-(Aminothiocarbonyl)-cyclohexylcarbonsäure-ethylester
Das Lösungsmittel wird unter vermindertem Druck eingedampft und der Rückstand mit Ether verrieben und abgenutscht. $R_f$-Wert: 0,29 (Kieselgel; Methylenchlorid/Methanol = 19:1)

Beispiel II

2-(4-Ethoxycarbonyl-phenyl)-4,5,6,7-tetrahydro-thiazolo-[5,4-c]pyridin-hydrobromid

Eine Lösung von 10,65 g 3-Brom-piperidin-4-on-hydrobromid und 8,6 g 4-(Aminothiocarbonyl)-benzoesäure-ethylester in 50 ml Dimethylformamid wird 9 Stunden auf 100 °C erwärmt. Anschließend wird das Lösungsmittel unter vermindertem Druck weitgehend abgedampft und das Rohprodukt zwischen Wasser und Essigester verteilt. Der sich zwischen den Phasen bildende Niederschlag wird abgesaugt und getrocknet.

Ausbeute: 3,0 g (20 % der Theorie),
$R_f$-Wert: 0,19 (Kieselgel; Methylenchlorid/Methanol = 9:1)

Die Essigesterphase wird abgetrennt und die wäßrige Phase mit Natriumhydrogencarbonat neutralisiert. Nach Extraktion der wäßrigen Phase mit Essigester wird die organische Phase getrocknet und das Lösungsmittel unter vermindertem Druck eingedampft. Man erhält weitere 1,4 g Produkt (12 % der Theorie) als freie Base.

Analog Beispiel II werden folgende Verbindungen erhalten:

(1) 2-(4-Ethoxycarbonyl-phenyl)-4,5,7,8-tetrahydro-6H-thiazolo[4,5-d]azepin
Es wird 5-Brom-azepin-4-on-hydrobromid eingesetzt. Die Lösung wird 3 Stunden auf 120 °C erwärmt. Anschließend wird mit Essigester verdünnt und der Niederschlag abgenutscht. Der Niederschlag wird in Wasser gelöst. Die wäßrige Lösung wird mit konz. Ammoniak bis zur alkalischen Reaktion versetzt und anschließend mit Essigester extrahiert. Die organische Phase wird getrocknet und das Lösungsmittel unter vermindertem Druck abgedampft.
$R_f$-Wert: 0,32 (Kieselgel; Methylenchlorid/Methanol/konz. Ammoniak = 8:2:0,2)
(2) 2-(3-Methoxycarbonyl-phenyl)-4,5,7,8-tetrahydro-6H-thiazolo[4,5-d]azepin
Es wird 5-Brom-azepin-4-on-hydrobromid eingesetzt. Die Lösung wird 3 Stunden auf dem Dampfbad erwärmt und die anschließende Aufarbeitung erfolgt analog Beispiel II(1).
$R_f$-Wert: 0,04 (Kieselgel; Methylenchlorid/Methanol/konz. Ammoniak = 9:1:0,1)
(3) 2-(1-Benzyloxycarbonyl-piperid-4-yl)-4,5,6,7-tetrahydro-thiazolo[5,4-c]pyridin
Das Rohprodukt wird über Kieselgel chromatographiert.
$R_f$-Wert: 0,18 (Kieselgel; Methylenchlorid/Methanol = 9:1)
(4) 2-(1-Benzyloxycarbonyl-piperid-4-yl)-4,5,7,8-tetrahydro-6H-thiazolo[4,5-d]azepin
Das Rohprodukt wird über Kieselgel chromatographiert.
$R_f$-Wert: 0,32 (Kieselgel; Methylenchlorid/Methanol/konz. Ammoniak = 9:1:0,1)
(5) 2-(4-Methoxycarbonyl-2-methyl-phenyl)-4,5,6,7-tetrahydro-thiazolo[5,4-c]pyridin-hydrobromid
$R_f$-Wert: 0,45 (Kieselgel; Methylenchlorid/Methanol/konz. Ammoniak = 9:1:0,1)
(6) 2-(4-Ethoxycarbonyl-cyclohexyl)-4,5,7,8-tetrahydro-6H-thiazolo[4,5-d]azepin
Es wird 5-Brom-azepin-4-on-hydrobromid eingesetzt. Die Aufarbeitung erfolgt analog Beispiel II(1).
$R_f$-Wert: 0,22 (Kieselgel; Methylenchlorid/Methanol/konz. Ammoniak = 9:1:0,1)

## Beispiel III

5-tert.Butyloxycarbonyl-2-(4-ethoxycarbonyl-phenyl)-4,5,6,7-tetrahydro-thiazolo[5,4-c]pyridin

Zu einer Suspension von 2,70 g 2-(4-Ethoxycarbonyl-phenyl)-4,5,6,7-tetrahydro-thiazolo[5,4-c]pyridin-hydrobromid und 1,0 ml Triethylamin in 100 ml Acetonitril gibt man 1,59 g Pyrokohlensäure-di-tert.butylester und rührt die Lösung 2 Stunden bei Raumtemperatur. Anschließend wird das Lösungsmittel unter vermindertem Druck eingedampft und der Rückstand zwischen Wasser und Essigester verteilt. Die Essigesterphase wird mit Wasser und verdünnter Kochsalzlösung gewaschen. Die organische Phase wird getrocknet und das Lösungsmittel unter vermindertem Druck abgedampft.
Ausbeute: 2,8 g (quantitativ),
$R_f$-Wert: 0,42 (Kieselgel; Methylenchlorid/Methanol = 19:1)
Das Produkt wird ohne Reinigung in Beispiel IV umgesetzt.

Analog Beispiel III werden folgende Verbindungen erhalten:

(1) 6-tert.Butyloxycarbonyl-2-(4-ethoxycarbonyl-phenyl)-4,5,7,8-thiazolo[4,5-d]azepin
$R_f$-Wert: 0,34 (Kieselgel; Methylenchlorid/Methanol = 30:1)
(2) 6-tert.Butyloxycarbonyl-2-(3-methoxycarbonyl-phenyl)-4,5,7,8-thiazolo[4,5-d]azepin
$R_f$-Wert: 0,40 (Kieselgel; Methylenchlorid/Methanol = 19:1)
(3) 5-tert.Butyloxycarbonyl-2-(4-methoxycarbonyl-2-methylphenyl)-4,5,6,7-tetrahydro-thiazolo[5,4-c]pyridin
$R_f$-Wert: 0,51 (Kieselgel; Methylenchlorid/Methanol = 19:1)
(4) 6-tert.Butyloxycarbonyl-2(4-ethoxycarbonyl-cyclohexyl)-4,5,7,8-thiazolo[4,5-d]azepin
Das Rohprodukt wird chromatographisch gereinigt. Man erhält ein Gemisch der cis/trans-Isomeren, welches in Beispiel IV(5) umgesetzt wird.
$R_f$-Wert: 0,51 und 0,62 (Kieselgel; Methylenchlorid/Methanol = 30:1; dreimalige Entwicklung)

## Beispiel IV

5-tert.Butyloxycarbonyl-2-(4-carboxy-phenyl)-4,5,6,7-tetrahydro-thiazolo[5,4-c]pyridin

Eine Lösung von 1,35 g 5-tert.Butyloxycarbonyl-2-(4-ethoxycarbonyl-phenyl)-4,5,6,7-tetrahydro-thiazolo-[5,4-c]pyridin und 3,8 ml einer 1.4 molaren Lithiumhydroxidlösung in 20 ml Tetrahydrofuran und 10 ml

Methanol wird 16 Stunden bei Raumtemperatur gerührt. Anschließend wird mit 5,3 ml 1N Salzsäure neutralisiert und mit Wasser verdünnt. Der Niederschlag wird abgenutscht. Durch Einengen der Mutterlauge unter vermindertem

Druck und nachfolgendem Abnutschen des Niederschlags wird weiteres Produkt erhalten.
Ausbeute: 1,20 g (87 % der Theorie),
$R_f$-Wert: 0,40 (Kieselgel; Methylenchlorid/Methanol = 9:1)

Analog Beispiel IV werden folgende Verbindungen erhalten:

(1) 6-tert.Butyloxycarbonyl-2-(4-carboxy-phenyl)-4,5,7,8-tetrahydro-thiazolo[4,5-d]azepin
$R_f$-Wert: 0,42 (Kieselgel; Methylenchlorid/Methanol = 9:1)
(2) 6-tert.Butyloxycarbonyl-2-(3-carboxy-phenyl)-4,5,7,8-tetrahydro-thiazolo[4,5-d]azepin
$R_f$-Wert: 0,46 (Kieselgel; Methylenchlorid/Methanol = 9:1)
(3) 2-(4-Carboxy-phenyl)-5-methyl-imidazo[4,5-d]azepin Nach dem Neutralisieren der Reaktionslösung mit 1N Salzsäure wird unter vermindertem Druck zur Trockne eingedampft. Der Rückstand wird in Wasser gelöst. Nach Zugabe von Methanol setzt Kristallisation ein.
$R_f$-Wert: 0,24 (Kieselgel; n-Butanol/Essigsäure/Wasser = 4:1:1)
(4) 5-tert.Butyloxycarbonyl-2-(4-carboxy-2-methyl-phenyl)-4,5,6,7-tetrahydro-thiazolo[5,4-c]pyridin
$R_f$-Wert: 0,38 (Kieselgel; Methylenchlorid/Methanol = 9:1)
(5) 6-tert.Butyloxycarbonyl-2-(trans-4-carboxy-cyclohexyl)-4,5,7,8-tetrahydro-thiazolo[4,5-d]azepin
Die Reaktionslösung wird mit Zitronensäure neutralisiert, überschüssiges Lösungsmittel wird unter vermindertem Druck entfernt und die wäßrige Phase mit Essigester extrahiert. Nach Trocknen der organischen Phase über Magnesiumsulfat wird das Lösungsmittel unter vermindertem Druck abgedampft und der Rückstand über Kieselgel chromatographiert.
$R_f$-Wert: 0,31 (Kieselgel; Methylenchlorid/Methanol = 19:1; dreimalige Entwicklung)

## Beispiel V

1-Benzyloxycarbonyl-piperid-4-yl-carbonsäureamid

Zu einer Lösung von 5,0 g Piperid-4-yl-carbonsäureamid und 5,54 ml Chlorameisensäurebenzylester in 30 ml Wasser und 40 ml Aceton tropft man langsam 39 ml 1N Natronlauge, so daß der pH-Wert zwischen pH 6-8 aufrechterhalten bleibt. Es wird eine Stunde bei Raumtemperatur gerührt. Anschließend wird das Aceton unter vermindertem Druck abgedampft und der Niederschlag abgenutscht und getrocknet.
Ausbeute: 8,8 g (86 % der Theorie),
$R_f$-Wert: 0,55 (Kieselgel; Methylenchlorid/Methanol/konz. Ammoniak = 9:1:0,1)

## Beispiel VI

1-Benzyloxycarbonyl-piperid-4-yl-thiocarbonsäureamid

Eine Lösung von 8,7 g 1-Benzyloxycarbonyl-piperid-4-yl-carbonsäureamid und 8,0 g 2,4-Bis-(4-methoxyphenyl)-1,3-dithia-2,4-diphosphetan-2,4-disulfid in 150 ml Tetrahydrofuran wird 4 Stunden bei Raumtemperatur gerührt. Das Lösungsmittel wird unter vermindertem Druck abgedampft und der Rückstand über Kieselgel chromatographiert.
Ausbeute: 7,7 g (83 % der Theorie),
$R_f$-Wert: 0,59 (Kieselgel; Methylenchlorid/Methanol = 9:1)

## Beispiel VII

2-(1-Benzyloxycarbonyl-piperid-4-yl)-6-trifluoracetyl-4,5,7,8-tetrahydro-thiazolo[4,5-d]azepin

Eine Lösung von 1,6 g 2-(1-Benzyloxycarbonyl-piperid-4-yl)-4,5,7,8-tetrahydro-6H-thiazolo[4,5-d]azepin, 0,9 ml Triethylamin und 0,9 ml Trifluoressigsäureanhydrid in 50 ml Methylenchlorid wird 16 Stunden bei Raumtemperatur gerührt. Anschließend wird die Lösung mit Wasser, mit 0,5N Salzsäure und mit verdünnter Natriumhydrogencarbonatlösung gewaschen. Die organische Phase wird über Magnesiumsulfat getrocknet und das Lösungsmittel unter vermindertem Druck abgedampft.
Ausbeute: 1,9 g (94 % der Theorie),

$R_f$-Wert: 0,35 (Kieselgel; Methylenchlorid/Methanol = 19:1)

Analog Beispiel VII wird folgende Verbindung erhalten:

(1) 2-(1-Benzyloxycarbonyl-piperid-4-yl)-5-trifluoracetyl-4,5,6,7-tetrahydro-thiazolo[5,4-c]pyridin
$R_f$-Wert: 0,64 (Kieselgel; Methylenchlorid/Methanol = 9:1)

Beispiel VIII

6-Trifluoracetyl-2-(piperid-4-yl)-4,5,7,8-tetrahydro-thiazolo[4,5-d]azepin-dihydrobromid

Eine Lösung von 1,9 g 2-(1-Benzyloxycarbonyl-piperid-4-yl)-6-trifluoracetyl-4,5,7,8-tetrahydro-thiazolo-[4,5-d]azepin in 10 ml Essigesäure und 10 ml 35%igem Bromwasserstoff in Essigsäure wird 3 Stunden bei 15°C gerührt. Man gibt Ether hinzu und dekantiert. Man wiederholt dies mehrmals und nutscht ab. Das Produkt wird im Exsikkator über Kaliumhydroxid getrocknet.
Ausbeute: 1,9 g hygroskopisches Produkt (94 % der Theorie),
$R_f$-Wert: 0,27 (Kieselgel; Methylenchlorid/Methanol/konz. Ammoniak = 8:2:0,2)

Analog Beispiel VIII werden folgende Verbindungen erhalten:

(1) 5-Trifluoracetyl-2-(1-piperid-4-yl)-4,5,6,7-tetrahydrothiazolo[5,4-c]pyridin-dihydrobromid
$R_f$-Wert: 0,38 (Kieselgel; Methylenchlorid/Methanol/konz. Ammoniak = 8:2:0,2)
(2) 6-Methyl-2-(piperid-4-yl)-4,5,7,8-tetrahydro-thiazolo[4,5-d]azepin-trihydrobromid
$R_f$-Wert: 0,24 (Kieselgel; Methylenchlorid/Methanol/konz. Ammoniak = 7:3:0,5)

Beispiel IX

2-(4-Methoxycarbonyl-phenyl)-1H-imidazo[4,5-c]pyridin

16,9 g Terephthalsäure-monomethylester und 10,3 g 3,4-Di-amino-pyridin in 50 ml Phosphoroxychlorid werden 16 Stunden bei 120°C gerührt. Man gibt weitere 50 ml Phosphoroxychlorid zu und rührt 8 Stunden bei 120°C. Das noch heiße, zähe Rohprodukt wird unter kräftigem Rühren in ein Eis-Wasser-Gemisch gegossen und der kristalline Niederschlag abgenutscht. Das Kristallisat wird mit verdünnter Ammoniaklösung verrührt und erneut abgenutscht (Kristallisat 1). Die Mutterlauge wird mit konz. Ammoniak neutralisiert (pH 7-8) und der Niederschlag abgenutscht (Kristallisat 2). Die vereinigten Kristallisate 1 und 2 werden mit Aceton verrieben und abgenutscht.
Ausbeute: 2,0 g (79 % der Theorie),
$R_f$ -Wert: 0,36 (Kieselgel; Methylenchlorid/Methanol = 9:1)

Beispiel X

2-(4-Carboxy-phenyl)-1H-imidazo[4,5-c]pyridin

23 g 2-(4-Methoxycarbonyl-phenyl)-1H-imidazo[4,5-c]pyridin in 200 ml 6N Salzsäure werden 4 Stunden auf 100°C erwärmt. Der Niederschlag wird über eine G2-Fritte abgenutscht, anschließend in Wasser suspendiert und mit konzentriertem Ammoniak alkalisch gestellt. Zur Entfernung von nicht löslichen Bestandteilen wird über Kieselgur abgenutscht und die wäßrige Lösung mit Essigsäure neutralisiert. Der kristalline Niederschlag wird abgenutscht, mit Wasser, Aceton und Ether gewaschen.
Ausbeute: 14,0 g (64 % der Theorie),
$R_f$-Wert: 0,33 (Reversed Phase RP18, Methanol/5%ige Natriumchloridlösung = 6:4)

Beispiel XI

2-(4-Methoxycarbonyl-phenyl)-5-methyl-5H-imidazo[5,4-c]pyridin

Zu 5,0 g 2-(4-Carboxy-phenyl)-1H-imidazo[4,5-c]pyridin in 200 ml Dimethylsulfoxid gibt man 2,2 g Kalium-tert.butylat und rührt eine Stunde bei Raumtemperatur. Man tropft 1,3 ml Methyljodid hinzu und rührt eine weitere Stunde. Anschließend gibt man 2,2 g Kalium-tert.butylat zu, rührt 30 Minuten bei

Raumtemperatur, tropft weitere 1,3 ml Methyljodid zu und rührt eine weitere Stunde. Die Suspension wird mit Wasser verdünnt. Das auskristallisierende Produkt wird abgenutscht und getrocknet.

Ausbeute: 2,5 g (47 % der Theorie),
$R_f$-Wert: 0,09 (Kieselgel; Essigester/Methanol = 9:1)

Beispiel XII

2-(1-Benzyloxycarbonyl-piperid-4-yl)-6-methyl-4,5,7,8-tetrahydro-thiazolo[4,5-d]azepin

Eine Lösung von 9,0 g 2-(1-Benzyloxycarbonyl-piperid-4-yl)-4,5,7,8-tetrahydro-6H-thiazolo[4,5-d]azepin und 1,25 g einer 33%igen wäßrigen Formaldehydlösung in 50 ml Essigsäure werden 30 Minuten unter Gasentwicklung auf 100 °C erwärmt. Anschließend wird überschüssige Essigsäure abgedampft und der Rückstand in Wasser gelöst. Die wäßrige Phase wird mit Natriumhydrogencarbonat neutralisiert und mit Essigester extrahiert. Die Essigester-Phase wird mit Wasser gewaschen, über Magnesiumsulfat getrocknet und das Lösungsmittel unter vermindertem Druck abgedampft. Man erhält ein Öl, welches ohne weitere Reinigung nach Beispiel VIII umgesetzt wird.

Ausbeute: 8,8 g (94 % der Theorie),
$R_f$-Wert: 0,45 (Kieselgel; Methylenchlorid/Methanol/konz. Ammoniak = 9:1:0,1)

Beispiel XIII

4-Cyano-3-methyl-benzoesäure-methylester

Eine Suspension von 25 g 4-Brom-3-methyl-benzoesäure-methylester und 11,2 g Kupfercyanid in 50 ml Dimethylformamid wird 16 Stunden zum Rückfluß erhitzt. Die Reaktionslösung wird auf Wasser gegossen und die wäßrige Phase mehrmals mit Essigester extrahiert. Die organische Phase wird mit Wasser gewaschen, über Magnesiumsulfat getrocknet und das Lösungsmittel unter vermindertem Druck eingedampft. Der Rückstand wird mit Methylenchlorid über Kieselgel chromatographiert.

Ausbeute: 6,2 g (31 % der Theorie),
$R_f$:Wert: 0,33 (Kieselgel; Cyclohexan/Essigester = 7:3)

Beispiel XIV

trans-4-Carbamoyl-cyclohexylcarbonsäure-ethylester

Eine Lösung von 10 g trans-4-Carboxy-cyclohexylcarbonsäure-ethylester in 30 ml Thionylchlorid wird 2 Stunden bei Raumtemperatur gerührt. Überschüssiges Thionylchlorid wird unter vermindertem Druck abgedampft und der Rückstand in Ether gelöst. Zu dieser Lösung tropft man bei O °C eine konzentrierte Ammoniaklösung. Der ausfallende Niederschlag wird abgenutscht, anschließend in Methylenchlorid gelöst und die Lösung über Magnesiumsulfat getrocknet. Das Lösungsmittel wird unter vermindertem Druck abgedampft.

Ausbeute: 9,0 g (90 % der Theorie),
$R_f$-Wert: 0,45 (Kieselgel; Methylenchlorid/Methanol/konz. Ammoniak = 9:1:0,1)

Beispiel XV

trans-4-Cyano-cyclohexylcarbonsäure-ethylester

Eine Lösung von 9,0 g trans-4-Carbamoyl-cyclohexylcarbonsäure-ethylester, 14,8 g Triphenylphosphin, 6,3 ml Triethylamin und 4,8 ml Tetrachlorkohlenstoff in 100 ml Chloroform wird 16 Stunden zum Rückfluß erhitzt. Es werden 11,0 g Triphenylphosphin zugesetzt und weitere 5 Stunden zum Rückfluß erhitzt. Das Lösungsmittel wird unter vermindertem Druck abgedampft und der Rückstand mit Methylenchlorid als Eluens über Kieselgel chromatographiert.

Ausbeute: 7,0 g (86 % der Theorie),
$R_f$-Wert: 0,44 (Kieselgel; Methylenchlorid)

Herstellung der Endverbindungen:

Beispiel 1

5-tert.Butyloxycarbonyl-2-[4-[(trans-4-methoxycarbonyl-cyclohexyl)-aminocarbonyl]-phenyl]-4,5,6,7-tetrahydro-thiazolo-[5,4-c]pyridin

Zu einer Suspension 2,50 g 5-tert.Butyloxycarbonyl-2-(4-carboxy-phenyl]-4,5,6,7-tetrahydro-thiazolo[5,4-c]pyridin in 50 ml Tetrahydrofuran gibt man 1,11 g N,N'-Carbonyl-diimidazol und rührt 3 Stunden bei Raumtemperatur. Anschließend gibt man eine Lösung von 1,34 g trans-4-Amino-cyclohexancarbonsäure-methylester-hydrochlorid und 1,0 ml Triethylamin in 10 ml Tetrahydrofuran zu und rührt 5 Tage bei Raumtemperatur. Das Lösungsmittel wird abgedampft und der Rückstand zwischen Wasser und Methylenchlorid verteilt. Die Methylenchlorid Phase wird mit Natriumhydrogencarbonat-Lösung und Wasser gewaschen, getrocknet und eingedampft. Der Rückstand wird mit Methylenchlorid/Methanol (19:1) über wenig Kieselgel filtriert. Das Filtrat wird eingeengt, der Feststoff mit Ether verrieben und abgenutscht.

Ausbeute: 2,60 g (75 % der Theorie),
$R_f$-Wert: 0,75 (Kieselgel; Methylenchlorid/Methanol = 9:1) Massenspektrum: $M^+$ = 499

Analog Beispiel 1 werden folgende Verbindungen erhalten:

(1) 5-tert.Butyloxycarbonyl-2-[4-[4-(methoxycarbonylmethyl)-piperidinocarbonyl]-phenyl]-4,5,6,7-tetrahydro-thiazolo[5,4-c]pyridin
Als Aminkomponente wird Piperid-4-ylessigsäure-methylesterhydrochlorid eingesetzt.
$R_f$-Wert: 0,66 (Kieselgel; Methylenchlorid/Methanol = 9:1) Massenspektrum: $M^+$ = 499

(2) 6-tert.Butyloxycarbonyl-2-[4-[(trans-4-methoxycarbonylcyclohexyl)-aminocarbonyl]-phenyl]-4,5,6,7-tetrahydro-thiazolo[4,5-d]azepin
$R_f$-Wert: 0,54 (Kieselgel; Methylenchlorid/Methanol = 9:1) Massenspektrum: $M^+$ = 513

(3) 6-tert.Butyloxycarbonyl-2-[3-[(trans-4-methoxycarbonylcyclohexyl)-aminocarbonyl]-phenyl]-4,5,7,8-tetrahydro-thiazolo[4,5-d]azepin
$R_f$-Wert: 0,64 (Kieselgel; Methylenchlorid/Methanol/konz. Ammoniak = 9:1:0,1)
Massenspektrum: $M^+$ = 513

(4) 2-[4-[4-(Methoxycarbonylmethyl)-piperidinocarbonyl]-phenyl]-1H-imidazo[4,5-c]pyridin
Als Aminkomponente wird Piperid-4-ylessigsäure-methylesterhydrochlorid eingesetzt.
$R_f$-Wert: 0,24 (Kieselgel; Methylenchlorid/Methanol = 9:1)

(5) 2-[4-[(trans-4-Methoxycarbonyl-cyclohexyl)-aminocarbonyl]-phenyl]-1H-imidazo[4,5-c]pyridin
$R_f$-Wert: 0,58 (Kieselgel; Methylenchlorid/Methanol/Essigsäure = 8:2:0,2)
Massenspektrum: $M^+$ = 378

(6) 2-[4-[(trans-4-Methoxycarbonyl-cyclohexyl)-aminocarbonyl]-phenyl]-5-methyl-imidazo[5,4-c]pyridin
$R_f$-Wert: 0,13 (Kieselgel; Methylenchlorid/Methanol = 9:1)
Massenspektrum: $M^+$ = 392

(7) 5-tert.Butyloxycarbonyl-2-[4-[(trans-4-methoxycarbonylcyclohexyl)-aminocarbonyl]-2-methyl-phenyl]-4,5,6,7-tetrahydro-thiazolo[5,4-c]pyridin
Schmelzpunkt: ab 155 °C (Sintern)
$R_f$-Wert: = 0,49 (Kieselgel; Methylenchlorid/Methanol = 9:1)

(8) 6-tert.Butyloxycarbonyl-2-[trans-4-[(trans-4-methoxycarbonyl-cyclohexyl)-aminocarbonyl]-cyclohexyl]-4,5,7,8-tetrahydro-thiazolo[4,5-d]azepin
Schmelzpunkt: ab 215 °C (Sintern)
$R_f$-Wert: = 0,66 (Kieselgel; Methylenchlorid/Methanol = 9:1)
Massenspektrum: $M^+$ = 519

Beispiel 2

2-[4-[(trans-4-Methoxycarbonyl-cyclohexyl)-aminocarbonyl]phenyl]-4,5,6,7-tetrahydro-thiazolo[5,4-c]pyridin-hydrochlorid

Durch eine Lösung von 1,0 g 5-tert.Butyloxycarbonyl-2-[4-[(trans-4-methoxycarbonyl-cyclohexyl)-aminocarbonyl]-phenyl]-4,5,6,7-tetrahydro-thiazolo[5,4-c]pyridin in 50 ml Dioxan leitet man 15 Minuten Chlorwasserstoff. Nach 16-stündigem Rühren bei Raumtemperatur gibt man soviel Methylenchlorid zu bis

der Niederschlag vollständig gelöst ist. Nach Zugabe von mit Chlorwasserstoff gesättigtem Ether wird erneut 2 Stunden bei Raumtemperatur gerührt. Anschließend wird das Lösungsmittel unter vermindertem Druck entfernt, der verbleibende Rückstand mit Ether verrieben und abgenutscht.

Ausbeute: 0,76 g (87 % der Theorie),
Schmelzpunkt: 290-300 °C (Zers.)

| | | | | | | | | |
|---|---|---|---|---|---|---|---|---|
| Ber.: | C | 57,85 | H | 6,01 | N | 9,64 | Cl | 8,13 |
| Gef.: | | 57,58 | | 6,27 | | 9,52 | | 8,05 |

$R_f$-Wert: 0,67 (Kieselgel; Methylenchlorid/Methanol = 8:2)
Massenspektrum: $M^+$ = 399

Analog Beispiel 2 werden folgende Verbindungen erhalten:

(1) 2-[4-[4-(Carboxymethyl)-piperidinocarbonyl]-phenyl]-4,5,6,7-tetrahydro-thiazolo[5,4-c]pyridin-hydrochlorid Es wird die Verbindung aus Beispiel 3(1) eingesetzt. Schmelzpunkt: 255-258 °C (Zers.)
$R_f$-Wert: 0,28 (Kieselgel; Methylenchlorid/Methanol/konz. Ammoniak = 8:2:0,2)
Massenspektrum: $M^+$ = 385

(2) 2-[4-[(trans-4-Methoxycarbonyl-cyclohexyl)-aminocarbonyl]-phenyl]-4,5,7,8-tetrahydro-6H-thiazolo[4,5-d]azepinhydrochlorid
Es wird die Verbindung aus Beispiel 1(2) eingesetzt. Die Umsetzung erfolgt in einem 1:1-Gemisch aus Methylenchlorid und mit Chlorwasserstoff gesättigtem Ether.
Schmelzpunkt: 312-316 °C (Zers.)
$R_f$-Wert: 0,33 (Kieselgel; Methylenchlorid/Methanol/konz. Ammoniak = 9:1:0,1)
Massenspektrum: $M^+$ = 413

(3) 2-[3-[(trans-4-Methoxycarbonyl-cyclohexyl)-aminocarbonyl]-phenyl]-4,5,7,8-tetrahydro-6H-thiazolo[4,5-d]azepin-hydrochlorid
Es wird die Verbindung aus Beispiel 1(3) eingesetzt. Die Umsetzung erfolgt in einem 1:1:0,5-Gemisch aus Methylenchlorid, Methanol und mit Chlorwasserstoff gesättigtem Ether.
Schmelzpunkt: 108-110 °C (Zers.)
$R_f$-Wert: 0,14 (Kieselgel; Methylenchlorid/Methanol = 9:1) Massenspektrum: $M^+$ = 413

(4) 2-[4-[(trans-4-Methoxycarbonyl-cyclohexyl)-aminocarbonyl]-2-methyl-phenyl]-4,5,6,7-tetrahydro-thiazolo[5,4-c]pyridin-dihydrochlorid
Es wird die Verbindung aus Beispiel 1(7) eingesetzt. Die Umsetzung erfolgt in einem 1:1:2-Gemisch aus Methylenchlorid/Methanol und mit Chlorwasserstoff gesättigtem Ether.
Schmelzpunkt: ab 250 °C (Zers.)
$R_f$-Wert: 0,40 (Kieselgel; Methylenchlorid/Methanol/konz. Ammoniak = 9:1:0,1)
Massenspektrum: $M^+$ = 413

Beispiel 3

5-tert.Butyloxycarbonyl-2-[4-[(trans-4-carboxy-cyclohexyl)-aminocarbonyl]-phenyl]-4,5,6,7-tetrahydro-thiazolo[5,4-c]pyridin

Eine Lösung von 0,60 g 5-tert.Butyloxycarbonyl-2-[4-[(trans-4-methoxycarbonyl)-aminocarbonyl]-phenyl]-4,5,6,7-tetrahydro-thiazolo[5,4-c]pyridin und 2,2 ml einer 1,4 normalen wäßrigen Lithiumhydroxidlösung in 10 ml Tetrahydrofuran und 10 ml Methanol wird 2 Tage bei Raumtemperatur gerührt. Anschließend wird mit 3 ml 1N Salzsäure neutralisiert und das Lösungsmittel unter vermindertem Druck partiell entfernt. Der Niederschlag wird abgenutscht und mit Wasser gewaschen. Der Feststoff wird in Tetrahydrofuran gelöst, die Lösung über Magnesiumsulfat getrocknet und das Lösungsmittel eingedampft.

Ausbeute: 0,40 g (69 % der Theorie),
$R_f$-Wert: 0,30 (Kieselgel; Methylenchlorid/Methanol = 9:1)
Massenspektrum: $(M-H)^-$ = 484

Analog Beispiel 3 werden folgende Verbindungen erhalten:

(1) 5-tert.Butyloxycarbonyl-2-[4-[4-(carboxymethyl)piperidinocarbonyl]-phenyl]-4,5,6,7-tetrahydro-thiazolo-[5,4-c]pyridin

$R_f$-Wert: 0,31 (Kieselgel; Methylenchlorid/Methanol = 9:1)

Massenspektrum: $(M-H)^-$ = 484

(2) 6-tert.Butyloxycarbonyl-2-[4-[(trans-4-carboxy-cyclohexyl)-aminocarbonyl]-phenyl]-4,5,7,8-tetrahydro-thiazolo[4,5-d]azepin

$R_f$-Wert: 0,27 (Kieselgel; Methylenchlorid/Methanol = 9:1)

Massenspektrum: $M^+$ = 499

(3) 2-[4-[(trans-4-Carboxy-cyclohexyl)-aminocarbonyl]-phenyl]-6-methyl-4,5,7,8-tetrahydro-thiazolo[4,5-d]-azepin Schmelzpunkt: > 320 °C (Zers.)

$R_f$-Wert: 0,62 (Kieselgel; Methylenchlorid/Methanol/konz. Ammoniak = 7:3:0,5)

Massenspektrum: $M^+$ = 413

(4) 2-[3-[(trans-4-Carboxy-cyclohexyl)-aminocarbonyl]-phenyl]-4,5,7,8-tetrahydro-6H-thiazolo[4,5-d]azepin

$R_f$-Wert: 0,27 (Kieselgel; Methylenchlorid/Methanol/konz. Ammoniak = 7:3:0,5)

Massenspektrum: $M^+$ = 399

(5) 2-[1-[(trans-4-Carboxy-cyclohexyl)-aminocarbonyl]-piperid-4-yl]-4,5,6,7-tetrahydro-thiazolo[5,4-c]-pyridin

Schmelzpunkt: 216-220 °C (Zers.)

$R_f$-Wert: 0,36 (Kieselgel; Methylenchlorid/Methanol/konz. Ammoniak = 7:3:0,3)

Massenspektrum: $M^+$ = 392

(6) 2-[1-[(trans-4-Carboxy-cyclohexyl)-aminocarbonyl]-piperid-4-yl]-4,5,7,8-tetrahydro-6H-thiazolo[4,5-d]-azepin Das Lösungsmittel wird unter vermindertem Druck abgedampft und der Rückstand über Kieselgel chromatographiert.

Schmelzpunkt: 248-252 °C (Zers.)

$R_f$-Wert: 0,22 (Kieselgel; Methylenchlorid/Methanol/konz. Ammoniak = 7:3:0,5)

Massenspektrum: $M^+$ = 406

(7) 2-[4-[4-(Carboxymethyl)-piperidinocarbonyl]-phenyl]-1H-imidazo[4,5-c]pyridin

$R_f$-Wert: 0,62 (Kieselgel; Methylenchlorid/Methanol/Essigsäure = 8:2:0,2)

Massenspektrum: $M^+$ = 364

(8) 2-[4-[(trans-4-Carboxy-cyclohexyl)-aminocarbonyl]-phenyl]-1H-imidazo[4,5-c]pyridin

$R_f$-Wert: 0,25 (Kieselgel; Methylenchlorid/Methanol/Essigsäure = 8:2:0,2)

Massenspektrum: $M^+$ = 364

(9) 2-[4-[(trans-4-Carboxy-cyclohexyl)-aminocarbonyl]-phenyl]-5-methyl-4,5,6,7-tetrahydro-imidazo[4,5-c]-pyridin Nach der Neutralisation mit 1N Salzsäure wird das Lösungsmittel unter vermindertem Druck abgedampft und der Rückstand chromatographiert.

$R_f$-Wert: 0,33 (Kieselgel; Methylenchlorid/Methanol/konz. Ammoniak = 6:4:0,4)

Massenspektrum: $M^+$ = 382

(10) 2-[4-[(trans-4-Carboxy-cyclohexyl)-aminocarbonyl]-phenyl]-5-methyl-imidazo[5,4-c]pyridin

$R_f$-Wert: 0,06 (Kieselgel; Methylenchlorid/Methanol/Essigsäure = 8:2:0,2)

Massenspektrum: $M^+$ = 378

(11) 2-[4-[(trans-4-Carboxy-cyclohexyl)-aminocarbonyl]-2-methyl-phenyl]-4,5,7,8-tetrahydro-6H-thiazolo-[4,5-d]azepin

(12) 2-[1-[(trans-4-Carboxy-cyclohexyl)-aminocarbonyl]-piperid-4-yl]-6-methyl-4,5,7,8-tetrahydro-thiazolo-[4,5-d]azepin

$R_f$-Wert: 0,44 (Kieselgel; Methylenchlorid/Methanol/konz. Ammoniak = 7:3:0,5)

Massenspektrum: $M^+$ = 420

(13) 2-[1-[N-(trans-4-Carboxy-cyclohexyl)-N-methyl-aminocarbonyl]-piperid-4-yl]-4,5,7,8-tetrahydro-6H-thiazolo[4,5-d]azepin

(14) 2-[4-[(trans-4-Carboxy-cyclohexyl)-methyloxy]-phenyl]-4,5,6,7-tetrahydro-thiazolo[5,4-c]pyridin

(15) 2-[trans-4-[(trans-4-Carboxy-cyclohexyl)-carbonylamino]-cyclohexyl]-4,5,7,8-tetrahydro-6H-thiazolo-[4,5-d]azepin

(16) 2-[trans-4-[(trans-4-Carboxy-cyclohexyl)-carbonylamino]-cyclohexyl]-4,5,6,7-tetrahydro-thiazolo[5,4-c]pyridin

(17) 2-[4-[N-Benzyl-N-(trans-4-carboxy-cyclohexyl)-aminocarbonyl]-piperidyl]-4,5,6,7-tetrahydro-thiazolo-[5,4-c]pyridin

(18) 2-[4-[(trans-4-Carboxy-cyclohexyl)-carbonylamino]-phenyl]-4,5,7,8-tetrahydro-6H-thiazolo[4,5-d]-azepin
(19) 2-[4-[(trans-4-Carboxy-cyclohexyl)-oxymethyl]-phenyl]-4,5,6,7-tetrahydro-thiazolo[5,4-c]pyridin
(20) 2-[2-[(trans-4-Carboxy-cyclohexyl)-aminocarbonyl]-pyrid-4-yl]-4,5,7,8-tetrahydro-6H-thiazolo[4,5-d]-azepin
(21) 2-[4-[4-(2-Carboxy-ethyl)-piperidinocarbonyl]-phenyl]-4,5,6,7-tetrahydro-thiazolo[5,4-c]pyridin
(22) 2-[trans-4-[(trans-4-Carboxy-cyclohexyl)-aminocarbonyl]-cyclohexyl]-1-methyl-imidazo[4,5-c]pyridin
(23) 2-[1-[(trans-4-Carboxy-cyclohexyl)-aminocarbonyl]-piperid-4-yl]-1-methyl-imidazo[4,5-c]pyridin
(24) 2-[1-[(trans-4-Carboxy-cyclohexyl)-aminocarbonyl]-piperid-4-yl]-1-methyl-4,5,7,8-tetrahydro-6H-imidazo[4,5-d]azepin
(25) 2-[trans-4-[(trans-4-Carboxy-cyclohexyl)-aminocarbonyl]-cyclohexyl]-1-methyl-4,5,6,7-tetrahydro-imidazo[4,5-c]pyridin
(26) 2-[4-[(trans-4-Carboxy-cyclohexyl)-carbonylamino]-phenyl]-4,5,7,8-tetrahydro-6H-1,3-oxazolo[4,5-d]-azepin
(27) 2-[4-[(trans-4-Carboxy-cyclohexyl)-aminocarbonyl]-2-fluor-phenyl]-4,5,7,8-tetrahydro-6H-thiazolo[4,5-d]azepin
(28) 2-[4-[(trans-4-Carboxy-cyclohexyl)-aminomethyl]-phenyl]-4,5,7,8-tetrahydro-6H-thiazolo[4,5-d]azepin
(29) 2-[4-[4-Carboxy-piperidinocarbonyl)-methyl]-phenyl]-4,5,6,7-tetrahydro-thiazolo[5,4-c]pyridin
(30) 2-[4-[2-(4-Carboxy-piperidino)-ethyl]-phenyl]-4,5,6,7-tetrahydro-thiazolo[5,4-c]pyridin
(31) 2-[trans-4-[(trans-4-Carboxy-cyclohexyl)-aminocarbonyl]-cyclohexyl]-4,5,6,7-tetrahydro-thiazolo[5,4-c]pyridin
(32) 2-[3-Brom-4-[(trans-4-carboxy-cyclohexyl)-carbonylamino]-phenyl]-4,5,7,8-tetrahydro-6H-thiazolo[4,5-d]azepin
(33) 2-[1-[(trans-4-Carboxy-cyclohexyl)-aminocarbonyl]-piperid-3-yl]-4,5,7,8-tetrahydro-6H-thiazolo[4,5-d]-azepin
(34) 5-Allyloxycarbonyl-2-[1-[(trans-4-carboxy-cyclohexyl)-aminocarbonyl]-piperid-4-yl]-4,5,6,7-tetrahydro-thiazolo[5,4-c]pyridin
(35) 6-Allyloxycarbonyl-2-[1-[(trans-4-carboxy-cyclohexyl)-aminocarbonyl]-piperid-4-yl]-4,5,7,8-tetrahydro-thiazolo[4,5-d]azepin
(36) 2-[4-[(trans-4-Carboxy-cyclohexyl)-aminocarbonyl]-piperazinyl]-4,5,6,7-tetrahydro-thiazolo[5,4-c]-pyridin
(37) 5-tert.Butyloxycarbonyl-2-[4-[(trans-4-carboxy-cyclohexyl)-aminocarbonyl]-2-methyl-phenyl]-4,5,6,7-tetrahydro-thiazolo[5,4-c]pyridin

Schmelzpunkt: ab 210 °C (Sintern)
$R_f$-Wert: 0,33 (Kieselgel; Methylenchlorid/Methanol = 9:1)

(38) 6-tert.Butyloxycarbonyl-2-[trans-4-[(trans-4-carboxy-cyclohexyl)-aminocarbonyl]-cyclohexyl]-4,5,7,8-tetrahydro-thiazolo[4,5-d]azepin

Schmelzpunkt: ab 260 °C (Sintern)
$R_f$-Wert: 0,44 (Kieselgel; Methylenchlorid/Methanol = 9:1)
Massenspektrum: $M^+$ = 505

## Beispiel 4

2-[4-[(trans-4-Carboxy-cyclohexyl)-aminocarbonyl]-phenyl]-4,5,6,7-tetrahydro-thiazolo[5,4-c]pyridin-trifluoracetat

Eine Lösung von 0,40 g 5-tert.Butyloxycarbonyl-2-[4-[(trans-4-carboxy-cyclohexyl)-aminocarbonyl]-phenyl]-4,5,6,7-tetrahydro-thiazolo[5,4-c]pyridin in 40 ml Methylenchlorid und 5 ml Trifluoressigsäure wird 2 Stunden bei Raumtemperatur gerührt. Anschließend wird das Lösungsmittel und die Trifluoressigsäure unter vermindertem Druck entfernt, der erhaltene Rückstand mit Ether verrieben und abgesaugt.

Ausbeute: 0,50 g (quantitativ),
Schmelzpunkt: 306-316 °C (Zers.)
$R_f$-Wert: 0,12 (Kieselgel; Methylenchlorid/Methanol = 8:2) Massenspektrum: $M^+$ = 385

Analog Beispiel 4 werden folgende Verbindungen erhalten:

(1) 2-[4-[(trans-4-Carboxy-cyclohexyl)-aminocarbonyl]-phenyl]-4,5,7,8-tetrahydro-6H-thiazolo[4,5-d]azepin-trifluoracetat

Schmelzpunkt: 300-308 °C (Zers.)
$R_f$-Wert: 0,11 (Kieselgel; Methylenchlorid/Methanol = 2:1)

| | | | | | | | | |
|---|---|---|---|---|---|---|---|---|
| Ber.:<br>Gef.: | C | 53,79<br>53,55 | H | 5,10<br>5,23 | N | 8,18<br>8,14 | S | 6,24<br>6,14 |

Massenspektrum: $(M+H)^+$ = 400

(2) 2-[1-[(trans-4-Carboxycyclohexyl)-aminocarbonyl]-piperid-4-yl]-4,5,6,7-tetrahydro-thieno[4,5-c]pyridin-trifluoracetat

(3) 2-[4-[(trans-4-Carboxy-cyclohexyl)-aminocarbonyl]-2-methyl-phenyl]-4,5,6,7-tetrahydro-thiazolo[5,4-c]-pyridin-trifluoracetat
Schmelzpunkt: 240-241 °C (Zers.),
$R_f$-Wert: 0,33 (Kieselgel; Methylenchlorid/Methanol/konz. Ammoniak = 7:3:0,5)

| | | | | | | | | |
|---|---|---|---|---|---|---|---|---|
| Ber.:<br>Gef.: | C | 53,79<br>53,71 | H | 5,10<br>5,32 | N | 8,18<br>8,07 | S | 6,24<br>6,40 |

Massenspektrum: $M^+$ = 399

(4) 2-[trans-4-[(trans-4-Carboxy-cyclohexyl)-aminocarbonyl]-cyclohexyl]-4,5,7,8-tetrahydro-6H-thiazolo-[4,5-d]azepin-trifluoracetat
Schmelzpunkt: ab 134 °C (Sintern),
$R_f$-Wert: 0,29 (Kieselgel; Methylenchlorid/Methanol/konz. Ammoniak = 7:3:0,5)
Massenspektrum: $M^+$ = 405

Beispiel 5

2-[4-[(trans-4-Methoxycarbonyl-cyclohexyl)-aminocarbonyl]-phenyl]-6-methyl-4,5,7,8-tetrahydro-thiazolo[4,5-d]azepin

Eine Suspension aus 300 mg 2-[4-[(trans-4-Methoxycarbonylcyclohexyl)-aminocarbonyl]-phenyl]-4,5,7,8-tetrahydro-6H-thiazolo[4,5-d]azepin, 80 mg Natriumcarbonat und 70 mg Kaliumacetat in 0,25 ml Ameisensäure und 0,12 ml einer 33%igen wäßrigen Formaldehydlösung werden 2 Stunden auf dem Dampfbad erhitzt. Nach Einengen im Vakuum wird der erhaltene Rückstand in Wasser gelöst und mit Natriumhydrogencarbonat bis zur alkalischen Reaktion versetzt. Nach Extraktion der wäßrigen Phase mit Essigester wird die organische Phase mit Wasser gewaschen, getrocknet und das Lösungsmittel unter vermindertem Druck entfernt.
Ausbeute: 80 mg (30 % der Theorie),
$R_f$-Wert: 0,53 (Kieselgel; Methylenchlorid/Methanol/konz. Ammoniak = 9:1:0,1)
Massenspektrum: $M^+$ = 427

Beispiel 6

2-[1-[(trans-4-Methoxycarbonyl-cyclohexyl)-aminocarbonyl]-piperid-4-yl]-6-trifluoracetyl-4,5,7,8-tetrahydro-thiazolo[4,5-d]azepin

Zu einer Lösung von 1,0 g Chlorameisensäure-4-nitro-phenylester und 0,95 g trans-4-Amino-cyclohexancarbonsäure-methylester-hydrochlorid in 50 ml Tetrahydrofuran tropft man bei -15 °C innerhalb von 30 Minuten eine Lösung von 1,68 ml Triethylamin in 10 ml Tetrahydrofuran. Man rührt eine Stunde bei -15 °C und tropft anschließend eine Lösung von 19 g 6-Trifluoracetyl-2-(piperid-4-yl)-4,5,7,8-tetrahydro-thiazolo[4,5-d]azepin-hydrobromid und 1,4 ml Triethylamin in 20 ml Tetrahydrofuran zu. Man läßt auf Raumtemperatur erwärmen und rührt 16 Stunden. Anschließend wird das Lösungsmittel unter vermindertem Druck entfernt und der erhaltene Rückstand zwischen Wasser und Essigester verteilt. Die organische Phase wird mit Wasser, 0,1N Ammoniaklösung und Wasser gewaschen und über Magnesiumsulfat getrocknet. Das Lösungsmittel wird unter vermindertem Druck abgedampft und der Rückstand über Kieselgel chromatographiert.
Ausbeute: 1,2 g (58 % der Theorie),

Schmelzpunkt: 167-169 °C
$R_f$-Wert: 0,44 (Kieselgel; Methylenchlorid/Methanol = 9:1) Massenspektrum: $M^+$ = 516

Analog Beispiel 6 werden folgende Verbindungen erhalten:

(1) 2-[1-[(trans-4-Methoxycarbonyl-cyclohexyl)-aminocarbonyl]-piperid-4-yl]-5-trifluoracetyl-4,5,6,7-tetrahydro-thiazolo[5,4-c]pyridin
$R_f$-Wert: 0,24 (Kieselgel; Methylenchlorid/Methanol = 19:1)
Massenspektrum: $M^+$ = 502

(2) 2-[1-[(trans-4-Methoxycarbonyl-cyclohexyl)-aminocarbonyl]-piperid-4-yl]-6-methyl-4,5,7,8-tetrahydro-thiazolo[4,5-d]azepin $R_f$-Wert: 0,31(Kieselgel; Methylenchlorid/Methanol = 9:1; 2x) Massenspektrum: $M^+$ = 434

(3) 6-Allyloxycarbonyl-2-[1-[(trans-4-methoxycarbonyl-cyclohexyl)-aminocarbonyl]-piperid-4-yl]-4,5,7,8-tetrahydro-thiazolo[4,5-d]azepin

(4) 5-Allyloxycarbonyl-2-[1-[(trans-4-methoxycarbonyl-cyclohexyl)-aminocarbonyl]-piperid-4-yl]-4,5,6,7-tetrahydro-thiazolo[5,4-c]pyridin

## Beispiel 7

2-[1-[(trans-4-Isobutyloxycarbonyl-cyclohexyl)-aminocarbonyl]-piperid-4-yl]-4,5,7,8-tetrahydro-6H-thiazolo-[4,5-d]azepin-hydrochlorid

Durch eine Lösung von 200 mg 2-[1-[(trans-4-Carboxy-cyclohexyl)-aminocarbonyl]-piperid-4-yl)-4,5,7,8-tetrahydro-6H-thiazolo[4,5-d]azepin in 20 ml Isobutanol und 5 ml Methylenchlorid leitet man bis zur Sättigung Chlorwasserstoff. Nach 2-tägigem Rühren bei Raumtemperatur gibt man Ether hinzu und nutscht den Niederschlag ab.
Ausbeute: 240 mg (91 % der Theorie),
Schmelzpunkt: 214-216 °C (Zers.)
$R_f$-Wert: 0,22 (Kieselgel; Methylenchlorid/Methanol = 8:2)
Massenspektrum: $M^+$ = 462

Analog Beispiel 7 werden folgende Verbindungen erhalten:

(1) 2-[1-[(trans-4-Methoxycarbonyl-cyclohexyl)-aminocarbonyl]-piperid-4-yl]-4,5,7,8-tetrahydro-6H-thiazolo[4,5-d]azepin-hydrochlorid
Die Umsetzung erfolgt in Methanol/Ether (4:1).
Schmelzpunkt: 205-208 °C (Zers.)
$R_f$-Wert: 0,25 (Kieselgel; Methylenchlorid/Methanol = 8:2)
Massenspektrum: $M^+$ = 420

(2) 2-[1-[(trans-4-Ethoxycarbonyl-cyclohexyl)-aminocarbonyl]-piperid-4-yl]-4,5,7,8-tetrahydro-6H-thiazolo-[4,5-d]azepin-hydrochlorid
Die Umsetzung erfolgt in Ethanol.
Schmelzpunkt: 200-210 °C (Zers.)
$R_f$-Wert: 0,25 (Kieselgel; Methylenchlorid/Methanol = 8:2)
Massenspektrum: $M^+$ = 434

(3) 2-[1-[(trans-4-Isopropoxycarbonyl-cyclohexyl)-aminocarbonyl]-piperid-4-yl]-4,5,7,8-tetrahydro-6H-thiazolo[4,5-d]azepin-hydrochlorid
Die Umsetzung erfolgt in Isopropanol.
Schmelzpunkt: 200-210 °C (Zers.)
$R_f$-Wert: 0,21 (Kieselgel; Methylenchlorid/Methanol = 8:2)
Massenspektrum: $M^+$ = 448

(4) 2-[1-[(trans-4-Ethoxycarbonyl-cyclohexyl)-aminocarbonyl]-piperid-4-yl]-4,5,6,7-tetrahydro-thiazolo[5,4-c]pyridin-hydrochlorid
Die Umsetzung erfolgt in Ethanol.
Schmelzpunkt: 210-241 °C (Zers.)
$R_f$-Wert: 0,36 (Kieselgel; Methylenchlorid/Methanol/konz. Ammoniak = 9:1:0,1)
Massenspektrum: $M^+$ = 420

(5) 2-[1-[(trans-4-Isobutyloxycarbonyl-cyclohexyl)-aminocarbonyl]-piperid-4-yl]-4,5,6,7-tetrahydro-thiazolo-[5,4-c]pyridin-hydrochlorid
Die Umsetzung erfolgt in Isobutanol.
Schmelzpunkt: 220-238 °C (Zers.)
$R_f$-Wert: 0,36 (Kieselgel; Methylenchlorid/Methanol/konz. Ammoniak = 9:1:0,1)
Massenspektrum: $M^+$ = 448

Beispiel 8

2-[4-[(trans-4-Methoxycarbonyl-cyclohexyl)-aminocarbonyl]phenyl]-5-methyl-4,5,6,7-tetrahydro-1H-imidazo-[4,5-c]pyridin

Eine Lösung von 740 mg 2-[4-[(trans-4-Methoxycarbonyl-cyclohexyl)-aminocarbonyl]-phenyl]-5-methyl-5H-imidazo[5,4-c)pyridin in 50 ml Essigsäure wird in Gegenwart von 0,4 g Platindioxid und einem Wasserstoffdruck von 5 bar 17 Stunden bei Raumtemperatur hydriert. Anschließend wird der Katalysator abgenutscht und die Lösung unter vermindertem Druck eingedampft. Der Rückstand wird mit Essigester verrieben und der Niederschlag abgenutscht.
Ausbeute: 650 mg (87 % der Theorie),
$R_f$-Wert: 0,16 (Kieselgel; Methylenchlorid/Methanol = 8:2)
Massenspektrum: $M^+$ = 396

Beispiel 9

5-Allyloxycarbonyl-2-[1-[[trans-4-[(pivaloyloxymethyl)-oxycarbonyl]-cyclohexyl]-aminocarbonyl]-piperid-4-yl]-4,5,6,7-tetrahydro-thiazolo[5,4-c]pyridin

Herstellung durch dreitägiges Rühren einer Suspension von 5-Allyloxycarbonyl-2-[1-[(trans-4-carboxy-cyclohexyl)-aminocarbonyl]-piperid-4-yl]-4,5,6,7-tetrahydro-thiazolo[5,4-c]pyridin, 2 Äquivalenten Pivalinsäure-chlormethylester, 2 Äquivalenten Kaliumiodid, 2 Äquivalenten Kaliumhydrogencarbonat und 2 Äquivalenten Kaliumcarbonat in Dimethylformamid bei Raumtemperatur.

Analog Beispiel 9 wird folgende Verbindung erhalten:

(1) 6-Allyloxycarbonyl-2-[1-[[trans-4-[[1-(ethoxycarbonyloxy)-ethyl]-oxycarbonyl]-cyclohexyl]-aminocarbonyl]-piperid-4-yl]-4,5,7,8-tetrahydro-thiazolo[4,5-d]azepin
Die Umsetzung wird mit 1-(Ethoxycarbonyloxy)-ethylchlorid in Dimethylsulfoxid durchgeführt.

Beispiel 10

2-[1-[[trans-4-[[1-(ethoxycarbonyloxy)-ethyl]-oxycarbonyl]-cyclohexyl]-aminocarbonyl]-piperid-4-yl]-4,5,7,8-tetrahydro-6H-thiazolo[4,5-d]azepin

Herstellung durch Zutropfen eines Äquivalents Morpholin zu 6-Allyloxycarbonyl-2-[1-[[trans-4-[[1-(ethoxycarbonyloxy)-ethyl]-oxycarbonyl]-cyclohexyl]-aminocarbonyl]-piperid-4-yl]-4,5,7,8-tetrahydro-thiazolo[4,5-d]-azepin und 0,1 Äquivalenten Tetrakis-(triphenylphosphin)-palladium(O) in Tetrahydrofuran und anschließendem einstündigem Rühren bei Raumtemperatur.

Analog Beispiel 10 wird folgende Verbindung erhalten:

(1) 2-[1-[[trans-4-[[pivaloyloxymethyl)-oxycarbonyl]-cyclohexyl]-aminocarbonyl]-piperid-4-yl]-4,5,6,7-tetrahydro-thiazolo[5,4-c]pyridin

Beispiel 11

Trockenampulle mit 2,5 mg Wirkstoff pro 1 ml

| Zusammensetzung: | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 12

Trockenampulle mit 35 mg Wirkstoff pro 2 ml

| Zusammensetzung: | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

Beispiel 13

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 14

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 15

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 16

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Bicyclische Heterocyclen der allgemeinen Formel

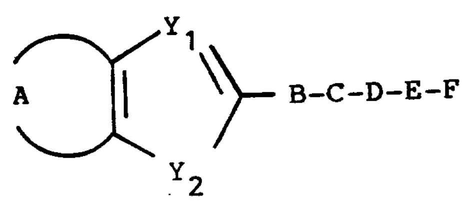

, (I)

in der

$Y_1$ ein Stickstoffatom oder ein durch den Rest $R_1$ substituiertes Kohlenstoffatom, wobei

$R_1$ ein Wasserstoffatom oder eine Alkylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine $-N=CH-NR_2-(CH_2)_m-$, $-(CH_2)_m-NR_2-CH=N-$, $-CH=CH-N=CH-$, $-CH=N-CH=CH-$ oder $-(CH_2)_n-NR_2-(CH_2)_p$-Brücke, welche im Kohlenstoffteil jeweils durch eine oder zwei Alkylgruppen substituiert sein kann, wobei

m die Zahl 1 oder 2,

n und p, die gleich oder verschieden sein können, jeweils die Zahl 1, 2 oder 3 und

$R_2$ ein Wasserstoffatom, eine Alkoxycarbonylgruppe mit insgesamt 2 bis 7 Kohlenstoffatomen, eine Alkyl-, Phenylalkyl-, Allyloxycarbonyl-, Phenylalkoxycarbonyl-, Trifluormethylcarbonyl-, $R_1CO$- oder $R_3CO-O-CHR_1-OCO$-Gruppe, wobei $R_1$ wie vorstehend erwähnt definiert ist und

$R_3$ eine Alkylgruppe mit 1 bis 5 Kohlenstoffatomen, eine Cycloalkylgruppe mit 5 bis 7 Kohlentstoffatomen, eine Phenylalkylgruppe oder eine Phenylgruppe bedeutet, darstellen,

B eine gegebenenfalls durch ein Fluor-, Chlor- oder Bromatom, durch eine Cyano-, Trifluormethyl-, Alkyl-, Hydroxy-, Alkoxy-, Alkylsulfenyl-, Alkylsulfinyl- oder Alkylsulfonylgruppe mono- oder disubstituierte Phenylengruppe, wobei die Substituenten gleich oder verschieden sein können und in der zusätzlich 1 oder 2 Methingruppen jeweils durch ein N-Atom ersetzt sein können, oder eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können, oder eine 1,4-Cyclohexylengruppe, in der die Methylengruppe in 2-Stellung oder die Methylengruppen in 2- und 5-Stellung jeweils durch eine $-NR_4$-Gruppe ersetzt sind, wobei in einem so erhaltenen Ring, der ein oder zwei Stickstoffatome oder eine oder zwei $-NR_4$-Gruppen enthält, jeweils eine oder zwei zu einem Stickstoffatom benachbarte Methylengruppen durch eine Carbonylgruppe ersetzt sein können, wobei jedoch jedem Ringstickstoffatom nur eine Carbonylgruppe benachbart sein kann, eine 3,4-Dehydro-1,4-piperidinylen- oder 1,3-Piperidinylengruppe, wobei

$R_4$ ein Wasserstoffatom, eine Alkyl- oder Phenylalkylgruppe darstellt,

C eine $-CH_2-$, $-CH_2CH_2-$, $-CO-$, $-CH_2-SO_2-$ oder $-SO_2-CH_2$-Gruppe, eine $-CH_2-CO-$ oder $-NR_4-CO-$Gruppe, wobei die Carbonylgruppe jeweils mit dem Rest D verknüpft sowie $R_4$ wie vorstehend erwähnt definiert ist,

eine $-CH_2-NR_4-$, $-NR_4-CH_2-$, $-O-CH_2-$, $-CH_2-O-$, $-S-CH_2-$, $-CH_2-S-$, $-SO-CH_2-$ oder $-CH_2-SO-$, wobei die Sulfinylgruppe, ein Stickstoff-, Sauerstoff- oder Schwefelatom nicht mit einem Stickstoffatom der Reste B oder D direkt oder über ein einzelnes Kohlenstoffatom verbunden sein kann und $R_4$ wie vorstehend erwähnt definiert ist,

D eine 1,4-Cyclohexylen- oder 1,4-Cyclohex-3-enylengruppe, in denen eine oder beide >CH-Einheiten, die mit den Resten C, E oder F verknüpft sind, durch ein Stickstoffatom ersetzt sein können und die >CH-Einheit, die mit dem Rest E oder F verknüpft ist, auch durch eine >C=CH-Gruppe ersetzt sein

kann, oder

eine $-NR_5-X$-Gruppe, in der

X eine geradkettige oder verzweigte Alkylengruppe mit 1 bis 5 Kohlenstoffatomen oder eine 1,4-Cyclohexylengruppe und

$R_5$ ein Wasserstoffatom, eine Phenylalkylgruppe mit 1 bis 4 Kohlenstoffatomen im Alkylteil oder eine Alkylgruppe mit 1 bis 6 Kohlenstoffatomen, welche in 1-, 2- oder 3-Stellung durch eine Morpholinocarbonyl-, Pyrrolidinocarbonyl-, Piperidinocarbonyl- oder Hexamethyleniminocarbonylgruppe oder durch eine $(R_6NR_7)$-CO-Gruppe substituiert sein kann, darstellen, wobei

$R_6$ ein Wasserstoffatom, eine Alkylgruppe mit 1 bis 5 Kohlenstoffatomen, eine Cycloalkylgruppe mit 5 bis 7 Kohlenstoffatomen, eine Phenyl- oder Phenylalkylgruppe und

$R_7$ ein Wasserstoffatom, eine Alkylgruppe mit 1 bis 4 Kohlenstoffatomen, eine Cyloalkylgruppe mit 5 bis 7 Kohlenstoffatomen oder eine Phenylgruppe darstellen,

E eine Alkylengruppe oder auch, falls D nicht über ein N-Atom mit dem Rest F direkt verbunden ist, eine Bindung und

F eine durch eine $R_8O$-Gruppe substituierte Carbonylgruppe, in der

$R_8$ ein Wasserstoffatom, eine Alkylgruppe mit 1 bis 6 Kohlenstoffatomen, die in 1-, 2-, 3-Stellung durch eine Morpholinocarbonyl-, Pyrrolidinocarbonyl-, Piperidinocarbonyl-, Hexamethyleniminocarbonyl- oder $(R_6NR_7)$-CO-Gruppe, wobei $R_6$ und $R_7$ wie vorstehend erwähnt definiert sind, oder in 2- oder 3-Stellung durch eine Morpholino- oder Pyrrolidinon-1-yl-Gruppe substituiert sein kann, eine Phenylalkyl- oder Pyridylalkylgruppe, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe mit 1 bis 4 Kohlenstoffatomen oder durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, eine Indanyl- oder Cinnamylgruppe darstellt,

eine Phosphono-, O-Alkylphosphono- oder $R_9CO-O-CHR_6-O-CO$-Gruppe darstellen, wobei

$R_6$ wie vorstehend erwähnt definiert ist und

$R_9$ eine Alkylgruppe mit 1 bis 5 Kohlenstoffatomen, eine Cycloalkyl- oder Cycloalkoxygruppe mit jeweils 5 bis 7 Kohlenstoffatomen im Cycloalkylteil, eine Alkoxygruppe mit

1 bis 4 Kohlenstoffatomen, eine Phenyl-, Phenylalkyl- oder Phenylalkoxygruppe darstellt,

wobei, soweit nichts anderes erwähnt wurde,

die vorstehend erwähnten Alkyl-, Alkylen- oder Alkoxyteile jeweils 1 bis 3 Kohlenstoffatome enthalten können,

deren Tautomere, deren Stereoisomere einschließlich deren Gemische und deren Salze.

**2.** Bicyclische Heterocyclen der allgemeinen Formel I gemäß Anspruch 1, in der

$Y_1$ ein Stickstoffatom oder ein durch den Rest $R_1$ substituiertes Kohlenstoffatom, wobei

$R_1$ ein Wasserstoffatom oder eine Alkylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine $-N=CH-NR_2-(CH_2)_m-$, $-(CH_2)_m-NR_2-CH=N-$, $-CH=CH-N=CH-$, $-CH=N-CH=CH-$ oder $-(CH_2)_n-NR_2-(CH_2)_p$-Brücke, welche im Kohlenstoffteil jeweils durch eine oder zwei Alkylgruppen substituiert sein kann, wobei

m die Zahl 1 oder 2,

n und p, die gleich oder verschieden sein können, jeweils die Zahl 1, 2 oder 3 und

$R_2$ ein Wasserstoffatom, eine Alkoxycarbonylgruppe mit insgesamt 2 bis 7 Kohlenstoffatomen, eine Alkyl-, Phenylalkyl-, Allyloxycarbonyl-, Phenylalkoxycarbonyl-, Trifluormethylcarbonyl-, $R_1CO$- oder $R_3CO-O-CHR_1-OCO$-Gruppe, wobei $R_1$ wie vorstehend erwähnt definiert ist und

$R_3$ eine Alkylgruppe mit 1 bis 5 Kohlenstoffatomen, eine Cycloalkylgruppe mit 5 bis 7 Kohlenstoffatomen, eine Phenylalkylgruppe oder eine Phenylgruppe bedeutet, darstellen,

B eine gegebenenfalls durch ein Fluor-, Chlor- oder Bromatom, durch eine Cyano-, Trifluormethyl-, Alkyl-, Hydroxy-, Alkoxy-, Alkylsulfenyl-, Alkylsulfinyl- oder Alkylsulfonylgruppe mono- oder disubstituierte Phenylengruppe, wobei die Substituenten gleich oder verschieden sein können und in der zusätzlich 1 oder 2 Methingruppen jeweils durch ein N-Atom ersetzt sein können, oder

eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können, oder eine 1,4-Cyclohexylengruppe, in der die Methylengruppe in 2-Stellung oder die Methylengruppen in 2- und 5-Stellung jeweils durch eine $-NR_4$-Gruppe ersetzt sind, wobei in einem so erhaltenen Ring, der ein

oder zwei Stickstoffatome oder eine oder zwei $-NR_4$-Gruppen enthält, jeweils eine oder zwei zu einem Stickstoffatom benachbarte Methylengruppen durch eine Carbonylgruppe ersetzt sein können, wobei jedoch jedem Ringstickstoffatom nur eine Carbonylgruppe benachbart sein kann, eine 3,4-Dehydro-1,4-piperidinylen- oder 1,3-Piperidinylengruppe, wobei

$R_4$ ein Wasserstoffatom, eine Alkyl- oder Phenylalkylgruppe darstellt,

C eine $-CH_2-$, $-CH_2CH_2-$, -CO-, $-CH_2-SO_2-$ oder $-SO_2-CH_2$-Gruppe, eine $-CH_2-CO-$ oder $-NR_4-CO-$Gruppe, wobei die Carbonylgruppe jeweils mit dem Rest D verknüpft sowie $R_4$ wie vorstehend erwähnt definiert ist,

eine $-CH_2-NR_4-$, $-NR_4-CH_2-$, $-O-CH_2-$, $-CH_2-O-$, $-S-CH_2-$, $-CH_2-S-$, $-SO-CH_2-$ oder $-CH_2-SO-$, wobei die Sulfinylgruppe, ein Stickstoff-, Sauerstoff- oder Schwefelatom nicht mit einem Stickstoffatom der Reste B oder D direkt oder über ein einzelnes Kohlenstoffatom verbunden sein kann und $R_4$ wie vorstehend erwähnt definiert ist,

D eine 1,4-Cyclohexylen- oder 1,4-Cyclohex-3-enylengruppe, in denen eine oder beide >CH-Einheiten, die mit den Resten C, E oder F verknüpft sind, durch ein Stickstoffatom ersetzt sein können und die >CH-Einheit, die mit dem Rest E oder F verknüpft ist, auch durch eine >C=CH-Gruppe ersetzt sein kann, oder

eine $-NR_5$-X-Gruppe, in der

X eine geradkettige oder verzweigte Alkylengruppe mit 1 bis 5 Kohlenstoffatomen oder eine 1,4-Cyclohexylengruppe und

$R_5$ ein Wasserstoffatom, eine Phenylalkylgruppe mit 1 bis 4 Kohlenstoffatomen im Alkylteil oder eine Alkylgruppe mit 1 bis 6 Kohlenstoffatomen, welche in 1-, 2- oder 3-Stellung durch eine Morpholinocarbonyl-, Pyrrolidinocarbonyl-, Piperidinocarbonyl- oder Hexamethyleniminocarbonylgruppe oder durch eine $(R_6NR_7)$-CO-Gruppe substituiert sein kann, darstellen, wobei

$R_6$ ein Wasserstoffatom, eine Alkylgruppe mit 1 bis 5 Kohlenstoffatomen, eine Cycloalkylgruppe mit 5 bis 7 Kohlenstoffatomen, eine Phenyl- oder Phenylalkylgruppe und

$R_7$ ein Wasserstoffatom, eine Alkylgruppe mit 1 bis 4 Kohlenstoffatomen, eine Cyloalkylgruppe mit 5 bis 7 Kohlenstoffatomen oder eine Phenylgruppe darstellen,

E eine Alkylengruppe oder auch, falls D nicht über ein N-Atom mit dem Rest F direkt verbunden ist, eine Bindung und

F eine durch eine $R_8O$-Gruppe substituierte Carbonylgruppe, in der

$R_8$ ein Wasserstoffatom, eine Alkylgruppe mit 1 bis 6 Kohlenstoffatomen, die in 1-, 2-, 3-Stellung durch eine Morpholinocarbonyl-, Pyrrolidinocarbonyl-, Piperidinocarbonyl-, Hexamethyleniminocarbonyl - oder $(R_6NR_7)$-CO-Gruppe, wobei $R_6$ und $R_7$ wie vorstehend erwähnt definiert sind, oder in 2- oder 3-Stellung durch eine Morpholino- oder Pyrrolidinon-1-yl-Gruppe substituiert sein kann, eine Phenylalkyl- oder Pyridylalkylgruppe, eine Cycloalkylgruppe mit 4 bis 8 Kohlenstoffatomen oder eine Cycloalkylalkylgruppe mit 3 bis 8 Kohlenstoffatomen im Cycloalkylteil, in denen jeweils die Cycloalkylgruppe durch eine Alkylgruppe mit 1 bis 4 Kohlenstoffatomen oder durch eine Alkylgruppe mit 1 bis 4 Kohlenstoffatomen und durch 1 bis 3 Methylgruppen substituiert sein kann, eine Bicycloalkyl- oder Bicycloalkylalkylgruppe, in denen der Bicycloalkylteil jeweils 6 bis 10 Kohlenstoffatome enthält und zusätzlich durch 1 bis 3 Methylgruppen substituiert sein kann, eine Indanyl- oder Cinnamylgruppe darstellt,

eine Phosphono-, O-Alkylphosphono- oder $R_9CO-O-CHR_6-O-CO$-Gruppe darstellen, wobei

$R_6$ wie vorstehend erwähnt definiert ist und

$R_9$ eine Alkylgruppe mit 1 bis 5 Kohlenstoffatomen, eine Cycloalkyl- oder Cycloalkoxygruppe mit jeweils 5 bis 7 Kohlenstoffatomen im Cycloalkylteil, eine Alkoxygruppe mit

1 bis 4 Kohlenstoffatomen, eine Phenyl-, Phenylalkyl- oder Phenylalkoxygruppe darstellt,

wobei, soweit nichts anderes erwähnt wurde,

die vorstehend erwähnten Alkyl-, Alkylen- oder Alkoxyteile jeweils 1 bis 3 Kohlenstoffatome enthalten können,

deren Tautomere, deren Stereoisomere einschließlich deren Gemische und deren Salze,

**3.** Bicyclische Heterocyclen der allgemeinen Formel I gemäß Anspruch 1, in der

$Y_1$ ein Stickstoffatom oder ein durch den Rest $R_1$ substituiertes Kohlenstoffatom, wobei

$R_1$ ein Wasserstoffatom, eine Methyl- oder Ethylgruppe darstellt,

$Y_2$ ein durch den Rest $R_1$ substituiertes Stickstoffatom, wobei $R_1$ wie vorstehend erwähnt definiert ist, ein Sauerstoff- oder Schwefelatom,

A eine -CH=CH-N=CH-, -CH=N-CH=CH-, $-(CH_2)_2-NR_2-CH_2-$ oder $-(CH_2)_2-NR_2-(CH_2)_2$-Brücke, in der

$R_2$ ein Wasserstoffatom, eine Methyl-, Ethyl-, Allyloxycarbonyl- oder Trifluormethylcarbonylgruppe oder eine Alkoxycarbonylgruppe mit insgesamt 2 bis 5 Kohlenstoffatomen darstellt,

B eine gegebenenfalls durch ein Fluor-, Chlor- oder Bromatom, durch eine Methyl- oder Ethylgruppe substituierte Phenylengruppe, in der zusätzlich eine Methingruppe durch ein N-Atom ersetzt sein kann, eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können, oder eine 1,3-Piperidinylengruppe,

C eine $-CH_2-$, $-CH_2CH_2-$ oder -CO-Gruppe,

eine $-CH_2$-CO- oder $-NR_4$-CO-Gruppe, wobei die Carbonylgruppe jeweils mit dem Rest D verknüpft und

$R_4$ ein Wasserstoffatom oder eine Alkylgruppe mit 1 oder 2 Kohlenstoffatomen darstellt,

eine $-CH_2-NR_4-$, $-NR_4-CH_2-$, $-O-CH_2-$ oder $-CH_2-O-$Gruppe, wobei das Stickstoff- oder Sauerstoffatom nicht mit einem Stickstoffatom der Reste B oder D direkt oder über ein einzelnes Kohlenstoffatom verbunden sein kann und $R_4$ wie vorstehend erwähnt definiert ist,

D eine 1,4-Cyclohexylengruppe, in der die >CH-Einheit in 1-Stellung, welche mit dem Rest C verknüpft ist, durch ein Stickstoffatom ersetzt sein kann, oder eine $-NR_5$-1,4-cyclohexylen-Gruppe, in der

$R_5$ ein Wasserstoffatom, eine Methyl-, Ethyl- oder Benzylgruppe darstellt,

E eine Alkylengruppe mit 1 bis 3 Kohlenstoffatomen oder eine Bindung und

F eine durch eine $R_8O$-Gruppe substituierte Carbonylgruppe oder eine $R_9CO-O-CHR_6-O-CO$-Gruppe bedeuten, in denen

$R_6$ ein Wasserstoffatom oder eine Methylgruppe,

$R_8$ ein Wasserstoffatom oder eine Alkylgruppe mit 1 bis 5 Kohlenstoffatomen und

$R_9$ eine Alkylgruppe mit 1 bis 5 Kohlenstoffatomen, eine Methoxy- oder Ethoxygruppe darstellen,

deren Tautomere, deren Stereoisomere einschließlich deren Gemische und deren Salze.

**4.** Bicyclische Heterocyclen der allgemeinen Formel I gemäß Anspruch 1, in der

$Y_1$ ein Stickstoffatom,

$Y_2$ eine -NH-Gruppe oder ein Schwefelatom,

A eine -CH=CH-N=CH-, $-(CH_2)_2-NR_2-CH_2-$ oder $-(CH_2)_2-NR_2-(CH_2)_2-$Brücke, in der

$R_2$ ein Wasserstoffatom, eine Methyl-, Ethyl- oder Trifluormethylcarbonylgruppe oder eine Alkoxycarbonylgruppe mit insgesamt 2 bis 5 Kohlenstoffatomen darstellt,

B eine gegebenenfalls durch eine Methylgruppe substituierte Phenylengruppe oder eine 1,4-Cyclohexylengruppe, in der eine >CH-Einheit in 1- oder 4-Stellung durch ein Stickstoffatom oder die >CH-Einheiten in 1- und 4-Stellung jeweils durch ein Stickstoffatom ersetzt sein können,

C eine -CO-Gruppe,

D eine 1,4-Cyclohexylengruppe, in der die >CH-Einheit in 1-Stellung, welche mit dem Rest C verknüpft ist, durch ein Stickstoffatom ersetzt sein kann, oder eine $-NR_5$-1,4-cyclohexylen-Gruppe, in der

$R_5$ ein Wasserstoffatom oder eine Methylgruppe darstellt,

E eine Methylengruppe oder eine Bindung und

F eine durch eine $R_8O$-Gruppe substituierte Carbonylgruppe, in der

$R_8$ ein Wasserstoffatom oder eine Alkylgruppe mit 1 bis 5 Kohlenstoffatomen darstellt,

deren Tautomere, deren Stereoisomere einschließlich deren Gemische und deren Salze.

**5.** Folgende Verbindungen der allgemeinen Formel I gemäß Anspruch 1:

(a) 2-[4-[(trans-4-Carboxy-cyclohexyl)-aminocarbonyl]-phenyl]-4,5,6,7-tetrahydro-thiazolo[5,4-c]-pyridin,

(b) 2-[4-[(trans-4-Methoxycarbonyl-cyclohexyl)-aminocarbonyl]-phenyl]-4,5,6,7-tetrahydro-thiazolo-[5,4-c]pyridin,

(c) 2-[4-[(trans-4-Carboxy-cyclohexyl)-aminocarbonyl]-phenyl]-4,5,7,8-tetrahydro-6H-thiazolo[4,5-d]-azepin,

(d) 2-[4-[(trans-4-Methoxycarbonyl-cyclohexyl)-aminocarbonyl]-phenyl]-4,5,7,8-tetrahydro-6H-thiazolo[4,5-d]azepin,

(e) 2-[1-[(trans-4-Carboxy-cyclohexyl)-aminocarbonyl]-piperid-4-yl]-4,5,6,7-tetrahydro-thiazolo[5,4-c]-pyridin,

(f) 2-[1-[(trans-4-Carboxy-cyclohexyl)-aminocarbonyl]-piperid-4-yl]-4,5,7,8-tetrahydro-6H-thiazolo[4,5-d]azepin,

(g) 2-[1-[(trans-4-Methoxycarbonyl-cyclohexyl)-aminocarbonyl]-piperid-4-yl]-4,5,7,8-tetrahydro-6H-thiazolo[4,5-d]azepin,

(h) 2-[1-[(trans-4-Isobutyloxycarbonyl-cyclohexyl)-aminocarbonyl]-piperid-4-yl]-4,5,7,8-tetrahydro-6H-thiazolo[4,5-d]azepin,

(i) 2-[4-[(trans-4-Carboxy-cyclohexyl)-aminocarbonyl]-2-methyl-phenyl]-4,5,6,7-tetrahydro-thiazolo-[5,4-c]pyridin,
(j) 2-[4-[(trans-4-Methoxycarbonyl-cyclohexyl)-aminocarbonyl]-2-methyl-phenyl]-4,5,6,7-tetrahydro-thiazolo[5,4-c]pyridin,
(k) 2-[trans-4-[(trans-4-Carboxy-cyclohexyl)-aminocarbonyl]-cyclohexyl]-4,5,7,8-tetrahydro-6H-thiazolo[4,5-d]azepin,
(1) 2-[1-[(trans-4-Ethoxycarbonyl-cyclohexyl)-aminocarbonyl]-piperid-4-yl]-4,5,7,8-tetrahydro-6H-thiazolo[4,5-d]azepin,
(m) 2-[1-[(trans-4-Isopropoxycarbonyl-cyclohexyl)-aminocarbonyl]-piperid-4-yl]-4,5,7,8-tetrahydro-6H-thiazolo[4,5-d]azepin
und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der bicyclischen Heterocyclen gemäß den Ansprüchen 1 bis 6, dadurch gekennzeichnet, daß
a) zur Herstellung von Verbindungen der allgemeinen Formel I, in der $R_2$ ein Wasserstoffatom oder F eine Carboxygruppe oder $R_2$ ein Wasserstoffatom und F eine Carboxygruppe darstellen, eine Verbindung der allgemeinen Formel

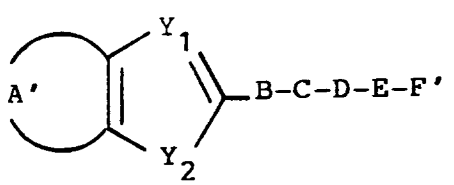

, (II)

in der
B bis E, $Y_1$ und $Y_2$ wie in den Ansprüchen 1 bis 5 definiert sind,
A' die für A in den Ansprüchen 1 bis 5 erwähnten Bedeutungen besitzt, wobei jedoch zusätzlich $R_2$ einen mittels Hydrolyse, Behandeln mit einer Säure oder Base, Thermolyse oder Hydrogenolyse abspaltbaren Rest darstellt, und F' eine Carboxygruppe oder eine mittels Hydrolyse, Behandeln mit einer Säure, Thermolyse oder Hydrogenolyse in eine Carboxygruppe überführbare Gruppe bedeutet, wobei jedoch mindestens einer der Reste A' oder F' eine mittels Hydrolyse, Behandeln mit Säure, Thermolyse oder Hydrogenolyse abspaltbaren Rest enthalten muß, in eine Verbindung der allgemeinen Formel I, in der $R_2$ ein Wasserstoffatom oder F eine Carboxylgruppe oder $R_2$ ein Wasserstoffatom und F eine Carboxygruppe darstellen, umgewandelt wird oder
b) zur Herstellung von Verbindungen der allgemeinen Formel I, in der C eine -CO- oder $-CH_2CO-$ Gruppe darstellt, eine Verbindung der allgemeinen Formel

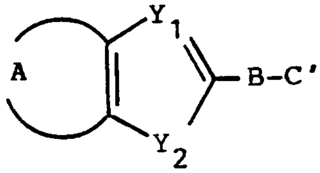

, (III)

in der
A, B, $Y_1$ und $Y_2$ wie in den Ansprüchen 1 bis 5 definiert sind und
C' eine Carboxy- oder Carboxymethylgruppe darstellt, mit einem Amin der allgemeinen Formel

H-D-E-F ,(IV)

in der
D bis F wie in den Ansprüchen 1 bis 5 definiert sind, umgesetzt wird oder
c) zur Herstellung von Verbindungen der allgemeinen Formel I, in der C eine -CO- oder -$NR_4$CO-Gruppe darstellt, eine Verbindung der allgemeinen Formel

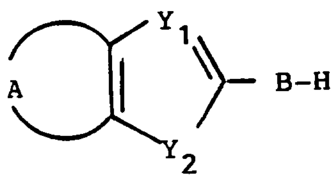

, (V)

in der
A, B, $Y_1$ und $Y_2$ wie in den Ansprüchen 1 bis 5 definiert sind, mit einer Verbindung der allgemeinen Formel

$Z_1$-C''-D-E-F ,(VI)

in der
D bis F wie in den Ansprüchen 1 bis 5 definiert sind, C'' eine Carbonylgruppe und
$Z_1$ eine Austrittsgruppe darstellen, umgesetzt wird oder
d) zur Herstellung von Verbindungen der allgemeinen Formel I, in der F eine durch eine $R_8$'O-Gruppe substituierte Carbonylgruppe darstellt, wobei $R_8$' mit Ausnahme des Wasserstoffatoms die für $R_8$ in den Ansprüchen 1 bis 5 erwähnten Bedeutungen besitzt, eine Verbindung der allgemeinen Formel

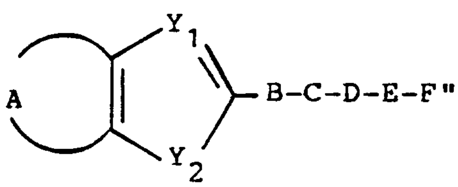

, (VII)

in der
A bis E, $Y_1$ und $Y_2$ wie in den Ansprüchen 1 bis 5 definiert sind und
F'' eine Carboxy- oder Alkoxycarbonylgruppe darstellt, mit einem Alkohol der allgemeinen Formel

HO - $R_8$' ,(VIII)

in der

$R_8$' mit Ausnahme des Wasserstoffatoms die für $R_8$ in den Ansprüchen 1 bis 5 erwähnten Bedeutungen besitzt, umgesetzt wird oder

e) zur Herstellung von Verbindungen der allgemeinen Formel I, in der F eine durch eine $R_8$'O-Gruppe substituierte Carbonylgruppe oder eine $R_9CO$-O-$CHR_6$-O-CO-Gruppe darstellt, wobei $R_6$ und $R_9$ wie in den Ansprüchen 1 bis 5 erwähnt definiert sind und $R_8$' mit Ausnahme des Wasserstoffatoms die für $R_8$ in den Ansprüchen 1 bis 5 erwähnten Bedeutungen besitzt, eine Verbindung der allgemeinen Formel

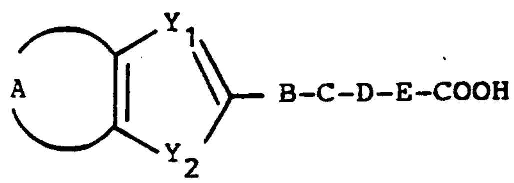

, (IX)

in der
A bis E, $Y_1$ und $Y_2$ wie in den Ansprüchen 1 bis 5 definiert sind, mit einer Verbindung der allgemeinen Formel

$Z_2$ - $R_{11}$ ,(X)

in der
$R_{11}$ mit Ausnahme des Wasserstoffatoms die für $R_8$ in den Ansprüchen 1 bis 5 erwähnten Bedeutungen besitzt oder eine $R_9CO$-O-$CHR_6$-Gruppe darstellt, wobei $R_6$ und $R_9$ wie in den Ansprüchen 1 bis 5 erwähnt definiert sind und
$Z_2$ eine Austrittsgruppe darstellt, umgesetzt wird oder

f) zur Herstellung von Verbindungen der allgemeinen Formel I, in der $R_2$ eine Alkoxycarbonylgruppe mit insgesamt 2 bis 5 Kohlenstoffatomen, eine Alkyl-, Phenylalkyl-, Allyloxycarbonyl-, Phenylalkoxycarbonyl-, Trifluormethylcarbonyl-, $R_1CO$-oder $R_3CO$-O-$CHR_1$-OCO-Gruppe darstellt, eine Verbindung der allgemeinen Formel

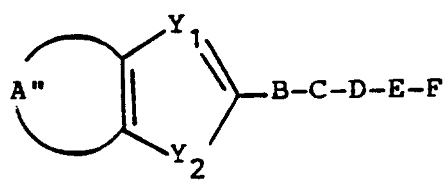

, (XI)

in der
B bis F, $Y_1$ und $Y_2$ wie in den Ansprüchen 1 bis 5 definiert sind und
A" eine $-N=CH-NH-(CH_2)_m-$, $-CH_2-NH-CH=N-$ oder $-(CH_2)_n-NH-(CH_2)_p-$Brücke, welche im Kohlenstoffteil jeweils durch eine oder zwei Alkylgruppen substituiert sein kann, wobei p, m und n wie in den Ansprüchen 1 bis 5 definiert sind, mit einer Verbindung der allgemeinen Formel

$Z_3$-$R_2$' ,(XII)

in der
$R_2$' mit Ausnahme des Wasserstoffatoms die für $R_2$ in den Ansprüchen 1 bis 5 erwähnten Bedeutungen besitzt und
$Z_3$ eine Austrittsgruppe oder
$Z_3$ zusammen mit einem benachbarten Wasserstoffatom des Restes $R_2$' ein Sauerstoffatom bedeuten, umgesetzt wird oder

g) zur Herstellung von Verbindungen der allgemeinen Formel I, in der A eine $-CH_2-NR_2-(CH_2)_2-$Brücke, in der $R_2$ ein Wasserstoffatom darstellt oder die für $R_{10}$ in den Ansprüchen 1 bis 5 erwähnten Bedeutungen besitzt und die im Kohlenstoffteil durch eine oder zwei Alkylgruppen substituiert sein kann, einer der Reste $Y_1$ oder $Y_2$ ein Stickstoffatom und der andere der Reste $Y_1$

oder $Y_2$ eine Iminogruppe darstellen, eine Verbindung der allgemeinen Formel

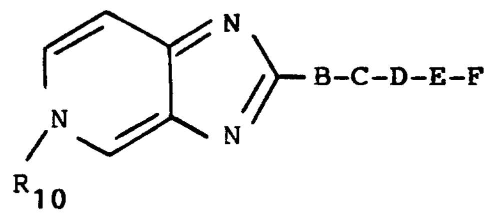

, (Ia)

in der
B bis F und $R_{10}$ wie in den Ansprüchen 1 bis 5 definiert sind, hydriert wird und
erforderlichenfalls ein während den Umsetzungen zum Schutze von reaktiven Gruppen verwendeter Schutzrest abgespalten wird und/oder
gewünschtenfalls eine so erhaltene Verbindung der allgemeinen Formel I in ihre Stereoisomere aufgetrennt wird und/oder eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

**11.** Zwischenprodukte der allgemeinen Formel

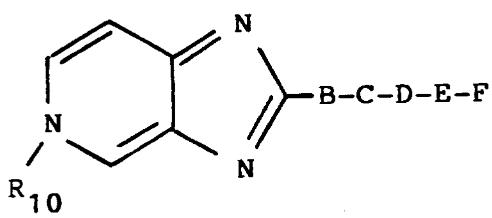

, (Ia)

in der
B bis F wie im Anspruch 1 definiert sind und
$R_{10}$ eine Alkyl- oder Phenylalkylgruppe mit jeweils 1 bis 3 Kohlenstoffatomen im Alkylteil bedeutet, und deren Salze.

Europäisches Patentamt

# EUROPÄISCHER RECHERCHENBERICHT

Nummer der Anmeldung
EP 94 11 1221

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 079 083 (THE WELLCOME FOUNDATION LIMITED) 18. Mai 1983<br>* Seite 5, Zeile 5 - Seite 5, Zeile 8; Ansprüche 1-16 *<br>--- | 1-9 | C07D471/04<br>C07D498/04<br>C07D513/04<br>A61K31/55<br>A61K31/435<br>//(C07D513/04, 211:00, 277:00), (C07D471/04, 277:00, 223:00), (C07D498/04, 263:00, 223:00), (C07D471/04, 213:00, 235:00), (C07D471/04, |
| Y | EP-A-0 407 217 (SCHERING CORPORATION) 9. Januar 1991<br>* Seite 7, Zeile 24 - Seite 8, Zeile 40; Ansprüche 1-10 *<br>--- | 1-9 | |
| P,Y | EP-A-0 581 166 (DR. KARL THOMAE GMBH) 2. Februar 1994<br>* Seite 21, Zeile 30 - Seite 21, Zeile 42; Ansprüche 1-8 *<br>--- | 1-9 | |
| P,Y | EP-A-0 560 330 (DR. KARL THOMAE) 15. September 1993<br>* Seite 16, Zeile 24 - Seite 16, Zeile 35; Ansprüche 1-9 *<br>--- | 1-9 | |
| Y | US-A-3 985 891 (BOEHRINGER INGELHEIM GMBH) 12. Oktober 1976<br>* Spalte 27, Zeile 5; Ansprüche 1-13 *<br>--- | 1-9 | **RECHERCHIERTE SACHGEBIETE (Int.Cl.6)**<br>C07D<br>A61K |
| P,Y | GB-A-2 264 115 (BRITISH BIO-TECHNOLOGY LIMITED) 18. August 1993<br>* Seite 24, Zeile 21 - Seite 25, Zeile 16; Ansprüche 1-18 *<br>--- | 1-9 | |
| Y | EP-A-0 351 856 (TAKEDA CHEMICAL INDUSTRIES, LTD.) 24. Januar 1990<br>* Seite 7, Zeile 32 - Seite 7, Zeile 58; Ansprüche 1-22,24-27 *<br>--- | 1-9 | |
| P,Y | EP-A-0 567 967 (DR. KARL THOMAE) 3. November 1993<br>* gesamtes Dokument *<br>---<br>-/-- | 1-9 | |

Der vorliegende Recherchenbericht wurde für alle Patentansprüche erstellt

| Recherchenort | Abschlußdatum der Recherche | Prüfer |
|---|---|---|
| MÜNCHEN | 19. Dezember 1994 | Herz, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung
EP 94 11 1221

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 296 455 (FUJISAWA PHARMACEUTICAL CO., LTD.) 28. Dezember 1988 * Seite 5, Zeile 52 - Seite 7, Zeile 15; Ansprüche 1-3,5-7 * | 1-9 | 223:00,235:00) |
| Y | DE-A-37 22 992 (DR. KARL THOMAE GMBH) 19. Januar 1989 * Seite 6, Zeile 5 - Seite 6, Zeile 8; Ansprüche 1-9 * | 1-9 | |
| Y | PATENT ABSTRACTS OF JAPAN vol. 13, no. 199 (C-594) 11. Mai 1989 & JP-A-01 019 084 (FUJISAWA PHARMACEUT. CO., LTD.) 23. Januar 1989 * Zusammenfassung * | 1-9 | |
| | | | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| MÜNCHEN | 19. Dezember 1994 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument